# EUROPEAN PATENT SPECIFICATION

(11) **EP 4 319 785 B1**
(45) Date of publication and mention of the grant of the patent: **09.09.2026**
(21) Application number: 22713755.1
(22) Date of filing: 28.03.2022
(51) Int. Cl.: A61K 36/638, A61K 8/9789, A61P 17/00, A61Q 19/08

(54) **LIGUSTRUM LUCIDUM EXTRACT USEFUL FOR THE TREATMENT AND/OR CARE OF THE SKIN**
FÜR DIE BEHANDLUNG UND/ODER PFLEGE DER HAUT NÜTZLICHER LIGUSTRUM-LUCIDUM-EXTRAKT
EXTRAIT DE LIGUSTRUM LUCIDUM UTILE POUR LE TRAITEMENT ET/OU LE SOIN DE LA PEAU

(30) Priority: 06.04.2021 EP 21382285
(43) Date of publication of application: 14.02.2024
(73) Proprietor: Lubrizol Advanced Materials, Inc., Cleveland, OH 44141-3247 (US)
(72) Inventor: ALMIÑANA DOMÈNECH, Núria, 08030 Barcelona (ES); VALERIO SANTIAGO, Mauricio, 11405 Cadiz (ES); VENKATARAMAN, Sylesh Kumar, Frisco, Texas 75035 (US); KIM, JungMo, Hurst, Texas 76054 (US)
(74) Representative: Bradley, Josephine Mary
(86) International application number: PCT/IB2022/052830
(87) International publication number: WO 2022/214908

(56) References cited:
- CN-A- 105 968 165
- CN-A- 107 281 273
- KR-A- 20200 009 195
- LIU JIA ET AL: "Bioactivity-guided isolation of immunomodulatory compounds from the fruits of Ligustrum lucidum", JOURNAL OF ETHNOPHARMACOLOGY, ELSEVIER IRELAND LTD, IE, vol. 274, 30 March 2021 (2021-03-30), XP086555800, ISSN: 0378-8741, [retrieved on 20210330], DOI: 10.1016/J.JEP.2021.114079
- SEO HYE LIM ET AL: "Liqustri lucidiFructus inhibits hepatic injury and functions as an antioxidant by activation of AMP-activated protein kinasein vivoandin vitro", CHEMICO-BIOLOGICAL INTERACTIONS, ELSEVIER SCIENCE IRLAND, IR, vol. 262, 1 December 2016 (2016-12-01), pages 57 - 68, XP029871703, ISSN: 0009-2797, DOI: 10.1016/J.CBI.2016.11.031
- LIN ET AL: "Protective effects of Ligustrum lucidum fruit extract on acute butylated hydroxytoluene-induced oxidative stress in rats", JOURNAL OF ETHNOPHARMACOLOGY, ELSEVIER IRELAND LTD, IE, vol. 111, no. 1, 29 March 2007 (2007-03-29), pages 129 - 136, XP022003924, ISSN: 0378-8741, DOI: 10.1016/J.JEP.2006.11.004
- MARCUS YIZHAK: "Extraction by Subcritical and Supercritical Water, Methanol, Ethanol and Their Mixtures", SEPARATIONS, vol. 5, no. 1, 1 January 2018 (2018-01-01), pages 4, XP055853921, DOI: 10.3390/separations5010004
- MASOUD GEORGINA N. ET AL: "HIF-1[alpha] pathway: role, regulation and intervention for cancer therapy", vol. 5, no. 5, 1 September 2015 (2015-09-01), pages 378 - 389, XP055853746, ISSN: 2211-3835, Retrieved from the Internet <URL:https://www.ncbi.nlm.nih.gov/pmc/articles/PMC4629436/pdf/main.pdf> DOI: 10.1016/j.apsb.2015.05.007

## Description

### FIELD OF THE INVENTION

The present invention relates to a botanical extract useful for the treatment and/or care of the skin. The botanical extract is obtainable from *Ligustrum lucidum* by extraction using subcritical water under specific conditions. The invention extends to the process for preparing the botanical extract, compositions comprising the botanical extract and the use of the botanical extract for the cosmetic, non-therapeutic treatment and/or care of the skin, as defined in the appended claims.

### BACKGROUND OF THE INVENTION

Skin is the largest organ in the human body and continuously requires an adequate amount of oxygen to perform its biological functions. Control of oxygen homeostasis in the skin is essential to maintain a proper hydration level, energetic status, production of extracellular matrix, and barrier function of the skin [Straseski, J.A. et al. "Oxygen deprivation inhibits basal keratinocyte proliferation in a model of human skin and induces regio-specific changes in the distribution of epidermal adherens junction proteins, aquaporin-3, and glycogen" (2009) Wound and Repair Regeneration 17(4):606-616; Steinbrech, D.S.].

Oxygen is delivered into the skin through two pathways: external delivery from atmospheric oxygen to the epidermis; and supply of oxygen through blood flow to the dermis. Atmospheric oxygen uptake takes place by diffusion in the few upper layers of the epidermis (0,25-0,4 mm depth). Basal layers of the epidermis remain in a relatively low or mild hypoxic state which is regulated by the expression of hypoxia induced factors (HIF) [Rosenberger, C. "Upregulation of Hypoxia-Inducible Factors in Normal and Psoriatic Skin" (2007) Journal of Investigative Dermatology. 127, 2445-2452]. Oxygen is supplied to the dermis from blood flow through cutaneous microcirculation (which supplies oxygenated hemoglobin to the skin in order to satisfy its oxygen demand). This process mainly depends on the vasoconstriction/ vasodilation activity of cutaneous microvessels.

The HIF-sensor system comprises oxygen-regulated transcription factors responsible for the control of tissue oxygenation. The family of HIF transcription factors includes three different isoforms (HIF-1α, HIF-2α and HIF-3α) which are targeted for degradation by HIF prolyl-hydroxylases under normoxia (normal levels of oxygen). However, hypoxic tissue conditions stabilize HIF factors, which bind to hypoxia response elements (HRE) in the DNA, leading to the upregulation of genes implicated in the improvement of oxygen delivery to the tissue [Brahimi-Horn, M.C. "HIF at a glance" (2009). Journal of Cell Science 122, 1055-1057]. This leads to the vasodilation of blood vessels.

The HIF-system plays an important role in the production of the vasodilation signal, increasing blood flow and oxygen supply to the hypoxic tissue. Nitric oxide is the best-known vasodilator in our body, contributing to 30-45% of cutaneous active vasodilation [Wong, BJ "Current concepts of active vasodilation in human skin" (2017) Temperature. 4:1, 41-59]. In epidermal cells, nitric oxide production is determined by the correct balance between the activity of the inducible nitric oxide synthase (iNOS) and the arginase enzyme. Both enzymes are controlled by different branches of the HIF-system: HIF-1α regulates iNOS expression, whereas HIF-2α controls arginase enzyme expression. Both enzymes compete for the same substrate, namely L-arginine. Depending on the equilibrium of the HIF isoforms expression level, the L-arginine is redirected to vasodilation signaling production by HIF-1α or to urea synthesis by HIF-2α. [Cowburn, A.S. "HIF isoforms in the skin differentially regulate systemic arterial pressure" (2013). Proceedings of the National Academy of Sciences 110(43); Andrew S Cowburn, A.S. "Cardiovascular adaptation to hypoxia and the role of peripheral resistance" (2017). Elife. 19;6]. Recently, it has been discovered that Tibetan population, who live in high altitude, where oxygen levels are low, has optimized oxygen delivery to their tissues by an adaptive mechanism. Tibetan population maintains an upregulated HIF-1α isoform in combination with a downregulation of the specific isoform HIF-2α under hypoxic conditions. A reduction in arginase activity in this population through the downregulation of HIF-2α isoform, increases L-arginine bioavailability in favor of the vasodilator production [Peng, Y "Down-Regulation of EPAS1 Transcription and Genetic Adaptation of Tibetans to High-Altitude Hypoxia" (2017). Molecular Biology and Evolution. 34(4):818-830; Song, D "Defective Tibetan PHD2 Binding to p23 Links High Altitude Adaption to Altered Oxygen Sensing" (2014) The Journal of biological Chemistry. 289:21, 14656 -14665].

Skin oxygenation decreases with age, leading to a pale facial appearance and acceleration of skin aging. This effect is more pronounced in older skin where the mechanisms involved in skin oxygenation are dysregulated. Particularly, mechanisms of vasodilation and vasoconstriction are altered during aging. There is an attenuation of vasodilation signals and an enhancement of the vasoconstriction due to an increase of arginase activity and the appearance of reactive oxygen species (ROS) during aging [Holowatz, L.A. "Aging and the control of human skin blood Flow" (2011) Frontiers in Bioscience. 15:718-739]. Impairment of HIF-1a function is also known to accelerate epidermal aging [Rezvani, HR "Loss of epidermal hypoxia-inducible factor-1a accelerates epidermal aging and affects re-epithelialization in human and mouse (2015). Journal of Cell Science. 124, 4172-4183].

Not surprisingly, there is a growing interest in cosmetic routines and practices directed to improve skin oxygenation. Beneficial effects such as improvement of facial skin color, healthier appearance [Stephen, ID. "Skin Blood Perfusion and Oxygenation Colour Affect Perceived Human Health" (2009). Plos One. 4(4): e5083], reduction of fine lines and better skin tone [Diaz, D "Oxygeneo®-A Unique Three-in-one Treatment of Exfoliation, Infusion, and Oxygenation via the Bohr Effect and TriPollar™ Radiofrequency for Skin Rejuvenation" (2017) Journal of Clinical and Aesthetic Dermatology. 10(11):22-25] have been reported for treatments improving skin oxygenation. Moreover, an upregulation of HIF-1 isoform has been associated with a skin rejuvenation strategy [Pagani, A. "Skin Rejuvenation through HIF-1alpha modulation" (2018). Plastic and Reconstructive Surgery Advance. 141(4):1].

WO2012045974A2 describes an active ingredient from *Tropaeolum majus* that includes arabinogalactanes for improving oxygenation of skin cells.

US20180028437A1 describes the use of a lipophilic extract of brown algae Undaria pinnatifida gameocytes as an anti-aging agent for the skin of the human body.

Endogenous reactive oxygen species (ROS) increase with aging and are capable of readily reacting with nitric oxide leading to the production of peroxynitrite, a reactive molecule with high vasoconstriction and cytotoxic effects [Holowatz, LA "Aging and the control of human skin blood Flow" (2011) Frontiers in Bioscience *15*:718-739]. Peroxynitrite can quickly oxidize some critical iNOS cofactors, which lead to a reduction of iNOS activity and, finally, nitric oxide production. In conclusion, strategies for enhancing vasodilator signals in the skin should not only rely on the production of nitric oxide, but also control other factors that can reduce its efficacy or may lead to undesirable side effects.

There is a need for treatments with higher efficacy in improving skin oxygenation and with less side effects.

*Ligustrum lucidum* (also commonly referred as glossy privet or Chinese privet) is an evergreen flowering plant in the olive family Oleaceae, native to the southern half of China and naturalized in many places. It is often used as an ornamental tree, but it has been widely used in traditional Chinese medicine over 1000 years, especially its fruit parts. The fruit of *Ligustrum lucidum* is known as Nu-zhen-zi in traditional Chinese medicine and are believed to nourish liver and kidney, treatment of tinnitus, vertigo, and soreness/weakness of the lower back and knees.

KR1896563B1 describes a cosmetic composition useful for moisturizing skin, comprising extracts from *L. lucidum* leaves. Preferred extraction solvents are water, 70% ethanol and butylene glycol and the preferred extraction temperature ranges are
18-80°C for water, and 18-40°C for EtOH and butylene glycol.

KR1901670B1 describes cosmetic compositions comprising extracts from *L*. *lucidum* for protecting skin from fine dust. In the examples, solvent extraction is carried out at room temperature using 70 % ethanol as an extraction solvent.

JP2013184942A describes extracts of *Ligustrum lucidum* or *Ligustrum japonicum* for providing antioxidant activity, inhibiting DNA damage and treating skin aging, in skin care compositions, and as cosmetics. The extract is obtained from the leaf or the fruit. In the example, extraction is carried out using 50 % ethanol at room temperature.

*L. lucidum* has also been studied as a potential anti-wrinkle cosmetic ingredient. Among the three parts of *L. lucidum* extracts (i.e., fruit, cane, and leaf extracts), the fruit extract was found to contain the greatest amounts of ursolic acid and oleanolic acid. The extract from the fruit is obtained with 95% aquous ethanol at 50-60 °C for 3 hours [Yong Deog Hong , "Excellent Anti-aging Effects of Ursolic acid and Oleanolic acid Present in Ligustrum lucidum" (2012) J. Soc. Cosmet. Scientists Korea Vol. 38, No. 2, 181-187].

KR2100345B1 describes cosmetic compositions comprising *Ligustrum lucidum* extracts with antioxidant, anti-inflammatory, and anti-wrinkle activity. In the example, extraction is carried out using 70 % ethanol at room temperature.

There is a need to find new active compounds of natural origin that can increase the oxygenation of skin, preferably with low side effects.

The present invention sets out to meet some or all of these needs and to solve some or all of the above-identified problems.

### SUMMARY OF THE INVENTION

The invention is set out in the appended claims.

The invention is based on the finding of a new botanical extract that can act advantageously in cosmetic applications. In particular, the extract is useful in the cosmetic non-therapeutic treatment and/or care of the skin, including: improving and/or increasing skin microcirculation or vascularization; improving or increasing skin oxygenation; improving or increasing skin rosiness (natural flush); reducing and/or preventing the symptoms of skin aging; increasing skin smoothness; and/or improving or increasing skin glossiness and/or luminosity. The botanical extract is an extract from the fruit of *Ligustrum lucidum* and can be obtained by solid-liquid extraction of the fruit of *Ligustrum lucidum* using subcritical water as an extraction solvent under specified conditions. The botanical extract can be obtained in the form of an aqueous botanical extract or a purified aqueous botanical extract.

A first apect the invention relates to a process for obtaining a botanical extract from fruit of *Ligustrum lucidum lucidium,* as defined in the appended claims.

In another aspect, the invention provides an aqueous botanical extract or a purified aqueous botanical extract obtainable from fruit of *Ligustrum lucidium,* as defined in the appended claims. The aqueous botanical extract can be purified by, for example, concentration or drying. The invention extends to the aqueous botanical extract so-purified.

In another aspect, the invention provides a composition comprising the aqueous botanical extract and a water-miscible organic solvent, or a composition comprising a purified aqueous botanical extract, a water-miscible organic solvent and, optionally, water. Each of these compositions can be useful as a stock solution, i.e. a solution in which the botanical extract is stored and which can be diluted to a lower concentration in use, for example, in use in cosmetic compositions.

In another aspect, the invention provides a cosmetic composition comprising: the aqueous botanical extract, the purified aqueous botanical extract, or one of the afore-mentioned compositions (comprising the aqueous botanical extract or the purified aqueous botanical extract and a water-miscible organic solvent); and a cosmetically acceptable excipient or ingredient.

In another aspect, the invention provides the use of the aqueous botanical extract or purified aqueous botanical extract for improving or increasing skin microcirculation and/or vascularization.

In another aspect, the invention provides the use of aqueous botanical extract or purified aqueous botanical extract for improving or increasing skin oxygenation.

In another aspect, the invention provides the use of the aqueous botanical extract or purified aqueous botanical extract for improving skin rosiness (natural flush).

In another aspect, the invention provides the use of the aqueous botanical extract or purified aqueous botanical extract for reducing and/or preventing the symptoms of skin aging.

In another aspect, the invention provides the use of the aqueous botanical extract or purified aqueous botanical extract for increasing skin smoothness.

In another aspect, the invention provides the use of the aqueous botanical extract or purified aqueous botanical extract for improving or increasing skin glossiness and/or luminosity.

In another aspect, the invention provides a method for the cosmetic, non-therapeutic treatment and/or care of the skin comprising topically administering an effective amount of the aqueous botanical extract or purified aqueous botanical extract to the skin. The cosmetic non-therapeutic treatment and/or care of the skin can be: the improvement and/or increase of skin microcirculation or vascularization; the improvement or increase of skin oxygenation; the improvement or increase of skin rosiness (natural flush); the reduction and/or prevention of the symptoms of skin aging; the increase of skin smoothness; and/or the improvement or increase skin glossiness and/or luminosity.

### DETAILED DESCRIPTION OF THE INVENTION

### Definitions

In the context of this invention "skin" is understood to be the layers which comprise it, from the uppermost layer or stratum corneum to the lowermost layer or hypodermis, both inclusive. These layers are composed of different types of cells such as keratinocytes, fibroblasts, melanocytes, mast cells, neurones and/or adipocytes among others. In the context of this invention, skin includes the skin of the whole body including the skin of the face (including skin around the eyes), neckline, neck, décolletage, arms, hands, legs, feet, thighs, hips, buttocks, stomach, torso and head scalp. The term "skin" includes the skin of mammals and includes human skin. Likewise, the terms "hair, nails and mucous membranes" include the hair, nails and mucous membranes of mammals, for example humans.

The term "treatment", as used herein and when it is not accompanied by the qualifications "cosmetic, non-therapeutic" refers to therapeutic methods including methods directed to the administration of a compound according to the invention to alleviate or eliminate a disease or disorder, or to reduce or eliminate one or more symptoms associated with said disease or disorder. The term "treatment", when it is not accompanied by the qualifications "cosmetic, non-therapeutic", also covers methods of therapy directed to alleviating or eliminating physiological consequences of the disease or disorder.

When the terms "treatment" and "care" are accompanied by the qualifications "cosmetic, non-therapeutic", it means that the treatment or care has the aim of improving or maintaining the aesthetic appearance of the skin, hair, nails and/or mucous membranes. In particular, the treatment can have the aim of improving cosmetic properties of the skin, hair, nails and/or mucous membranes such as, for example and not restricted to, the level of hydration, elasticity, firmness, shine, tone or texture, which properties affect the aesthetic appearance of the skin, hair, nails and/or mucous membranes. The term "care" in the context of this specification refers to the maintenance of properties of the skin, hair, nails and/or mucous membranes. Said properties are subject to being improved or maintained by cosmetic treatment and/or care of the skin, hair, nails and/or mucous membranes both in healthy subjects as well as in those which present diseases and/or disorders of the skin, hair, nails and/or mucous membranes.

The term "prevention", as used in this invention, refers to the ability of a compound of the invention to prevent, delay or hinder the appearance or development of a disease or disorder, or to prevent, delay or hinder the change in a cosmetic property of the skin, mucous membranes and/or hair. The term "prevention", as used in this invention, is interchangeable with the term "inhibition", i.e. it refers to the ability of a compound of the invention to inhibit the appearance or development of a disease or disorder, or to inhibit the change in a cosmetic property of the skin, hair, nails and/or mucous membranes.

In the context of this invention, the term "aging" refers to the changes experienced by the skin as the result of intrinsic aging process (i.e. chronoaging) or extrinsic skin aging process induced by environmental factors (i.e. through exposure to the sun (photoaging) or to environmental agents such as tobacco smoke, extreme climatic conditions of cold or wind, chemical contaminants or pollutants). In the context of the invention, aging includes all the external visible and/or perceptible changes through touch, such as and not restricted to, the development of discontinuities on the skin such as wrinkles, fine lines, expression lines, stretch marks, furrows, irregularities or roughness, increase in the size of pores, loss of hydration, loss of elasticity, loss of firmness, loss of smoothness, loss of the capacity to recover from deformation, loss of resilience, sagging of the skin such as sagging cheeks, the appearance of bags under the eyes or the appearance of a double chin, among others, changes to the color of the skin such as marks, reddening, bags or the appearance of hyperpigmented areas such as age spots or freckles among others, anomalous differentiation, hyperkeratinization, elastosis, keratosis, hair loss, orange-peel skin, loss of collagen structure and other histological changes of the stratum corneum, of the dermis, epidermis, vascular system (for example the appearance of spider veins or telangiectasias) or of those tissues close to the skin, among others. The term "photoaging" groups together the set of processes due to the prolonged exposure of the skin to ultraviolet radiation which result in the premature aging of the skin, and it presents the same physical characteristics as aging, such as and not restricted to, flaccidity, sagging, changes to the color or irregularities in the pigmentation, abnormal and/or excessive keratinization. The sum of various environmental factors such as exposure to tobacco smoke, exposure to pollution, and climatic conditions such as cold and/or wind also contribute to the aging of the skin.

The term "about" as used herein, e.g. when referring to a measurable value (such as an amount or weight of a particular component or temperature), refers to variations of ±20%, ±10%, ±5%, ±1%, ±0.5%, or, particularly, ±0.1% of the specified amount.

As used herein, the term "comprising", which is inclusive or open-ended and does not exclude additional unrecited elements or method steps, is intended to encompass as alternative embodiments, the phrases "consisting essentially of" and "consisting of" where "consisting of" excludes any element or step not specified and "consisting essentially of" permits the inclusion of additional unrecited elements or steps that do not materially affect the essential or basic and novel characteristics of the composition or method under consideration.

"Botanical extract" is understood to mean product obtained from a solid/liquid extraction, by means of which phytochemical compounds contained in a plant part (solid body) are solubilised by a solvent. Botanical extracts are also referred herein as "plant extracts".

### Process for obtaining a botanical extract from fruit of Ligustrum lucidum

In a first apect the invention provides a process for obtaining a botanical extract from fruit of *Ligustrum lucidum* comprising subjecting fruit of *Ligustrum lucidum* to an extraction with subcritical water, wherein the extraction comprises a step of contacting the fruit with subcritical water at a temperature of from 140°C to 180°C and at a pressure suitable to maintain the water in a liquid state for a period of at least 5 minutes so as to form an aqueous botanical extract.

The process involves a solid-liquid extraction from the fruit of *Ligustrum lucidum. Ligustrum lucidum* is a small tree from Oleacea family native to China and transported to different countries worldwide. *L. lucidum* is also commonly known as broad-leaf privet, Chinese privet, glossy privet, tree privet or wax-leaf privet. The fruit of *Ligustrum lucidum* it is also known as Nü-zhe-zi in China. Typically the fruit is harvested when ripe and dried for later use.

The process can involve a step of providing fruit of *Ligustrum lucidum* and thus the invention provides a process for obtaining a botanical extract from fruit of *Ligustrum lucidum* comprising: providing fruit of *Ligustrum lucidum;* and subjecting the fruit of *Ligustrum lucidum* to an extraction with subcritical water, wherein the extraction comprises a step of contacting the fruit with subcritical water at a temperature of at least 140°C and at a pressure suitable to maintain the water in a liquid state for a period of at least 5 minutes to form an aqueous botanical extract.

Typically, the fruit of *Ligustrum lucidum* are in dried form. The fruit can be dried so that they are easier to homogenize, manipulate and store. By dried fruit or fruit in dried form is meant fruit that has been dried so as to have a water content of less than 10% of water by weight, less than 5% of water by weight, less than 2% of water by weight or less than 1% of water by weight. This can be measured by AOAC Official Method AOAC 934.06-1934(1996), for example. However, fruit that has not been dried, such as ripe fruit may also be used. The fruit is also referred to herein as raw material.

Typically, the ratio of dried fruit:subcritical water (weight of dried fruit in g:volume of subcritical water in mL) used in the extraction is in a range of from 1:5 to 1:50, or from 1:10 to 1:30 or from 1:10 to 1:25 or from 1:15 to 20 g/mL or from 1:25 to 1.30 g/mL. Particularly, the ratio is 1:13, or 1:15, or 1:20, or 1:22 or 1:25 or 1:27 g/mL.

Advantageously, the fruit are ground or crushed before extraction. Ground fruit can have, for example, a particle size ranging from 100 pm to 50 mm, with an average particle size of from 0.5 to 5 mm. More particularly, the average particle size ranges from 0.5 to 1 mm. The average particle size can be determined by conventional methods such as those involving sieve analysis, for example. Any suitable grinding/crushing technique known in the art may be used to obtain the desired particle size of the fruit.

Preferably, the extraction is carried out on only the fruit of *Ligustrum lucidum,* i.e. no other plant material is present. The fruit can be mixed with a neutral material, to make it drier and/or more porous for solvent extraction. Suitable neutral materials include graphene, silica gel, C18-resins, diatomaceous earth and neutral alumina. A particularly suitable neutral material is diatomaceous earth.

The extraction solvent is subcritical water that is at a temperature of from 140°C to 180°C and held at a pressure that keeps it in a liquid state. Subcritical water is water that is held in the liquid state by pressure at a temperature higher than its natural boiling point of 100°C (i.e. it's boiling point at atmospheric pressure). Subcritical water can have a temperature up to its critical point temperature of 374°C. Subcritical water is also referred as "pressurized low polarity water", "pressurized hot water" or "compressed hot water". Heating water under pressure to temperatures above its boiling point results in the alteration of its key properties such as polarity. The extraction is carried out in a pressurizable container, typically a stainless steel container. Preferably, the only extraction solvent used in the process is subcritical water, i.e., the extraction is performed with subcritical water in the absence of any other organic or inorganic solvent.

The temperature of the subcritcal water is from 140°C to 180°C, and can be from 150°C to 175°C, or from 155°C to 170°C, or from 155°C to 165°C, or from 160°C to 165°C. Advantageously, the temperature is 160°C. During the extraction, the pressure in the pressurizable container must be such that the subcritical water is maintained in a liquid state. The pressure required to achieve this will vary depending on the temperature of the water. The skilled person would be able to determine the pressure required. Typically, the pressure will range from 0.5 MPa to 20 MPa. The pressure can be at least about 1 MPa. The pressure can be from 1 to about 5 Mpa or from 1 to 2 MPa.

The step of contacting the fruit with subcritical water is carried out for a period of at least 5 minutes. This period of time is the time that the fruit is in contact with subcritical water, whether the water is static or flowing over the fruit. This period is also referred to herein as the extraction time. The extraction time will vary, for example, depending on the amount of fruit and water used. Typically, the extraction time will vary from 5 minutes to about 5 hours. For example, the extraction time can be at least 15 minutes, or or at least 30 minutes, or at least 1 hour or at least 2 hours, or at least about 3 hours.

The extraction can be performed in batch mode (also referred as 'static mode') or in dynamic mode (also referred as 'flow-though'). In batch mode, the raw material (the fruit) is exposed to (i.e. contacted with) the subcritical water in batches. In batch mode, the volume of subcritical water used is the total volume of subcritcal water used for all the batches for the sample of raw material. In batch mode, the extraction time is the total time that the raw material is exposed to the subcritical water over all the batches for the sample of raw material. In dynamic mode, the raw material is exposed to a continuous flow of water. In dynamic mode, the volume of subcritical water used is the total volume of subcritcal water used for the sample of raw material. In dynamic mode, the extraction time is the total time that the raw material is exposed to the flow of subcritical water for the sample of raw material. For faster, larger scale production of the botanical extract, advantageously, the extraction is performed in dynamic mode.

When the extract is obtained in batch mode the extraction time may range from about 5 to 45 minutes, from about 5 to 30 minutes, or from about 10 to 20 minutes. The extraction time can be about 15 minutes.

When the extract is obtained in dynamic mode, the extraction time may ranges from about 1 hour to about 5 hours, or from about 2 hours to about 4 hours. The extraction time can be from about 3 hours to about 3.5 hours. In dynamic mode, the flow rate of the subcritical water can be from about 5 to 30 mL/min. The flow rate can be from about 10 to 20 mL/min, or from about 14 to 16 mL/min.

During the the step of contacting the fruit with subcritical water, components are extracted from the fruit into the subcritical water. More specifically, components of the fruit dissolve in the subcritical water thus forming an aqueous botanical extract. Compositional details of the aqueous botanical extract are described in the section below entitled "Botanical extract obtained from *Ligustrum lucidum".* The process comprises a step of separating the fruit (i.e. any undissolved fruit) from the aqueous botanical extract. Typically, this step is straightforward and simply involves releasing the aqueous botanical extract from the pressurized container while retaining the fruit in the pressurize container. Other suitable separation techniques are known in the art and include, for example, filtration, sedimentation, decantation or centrifugation. Filtration can be carried out using filters having a pore size of lower than 1000 µm, or lower than 20 µm, or lower than 10 µm, or lower than 1 µm, or lower than 0.1 µm. Filtration may be performed in successive filtration operations, for instance using filters of decreasing pore size. The residue (undissolved fruit) left after filtration may be re-contacting with more extraction solvent. This filtration-recontacting step may be carried out once or may be repeated, for example, repeated 1 to 5 times. Preferably, separation is carried out before the aqueous botanical extract cools and precipitates extract components.

After the aqueous botanical extract has been separated from the fruit, it can be purified (to form a "purified aqueous botanical extract"). In the context of this invention, "purifying" means purification, partial purification, and/or fractionation. There are a large number of techniques well known in the art for the purification of botanical extracts. Some non-limiting examples include solid-liquid extraction, liquid-liquid extraction, solid-phase extraction (SPE), membrane filtration, ultrafiltration, dialysis, electrophoresis, solvent concentration, centrifugation, ultracentrifugation, liquid or gas phase chromatography (including size exclusion, affinity, etc.) with or without high pressure, lyophilisation, evaporation, precipitation with various "carriers" (including PVPP, carbon, antibodies, etc.), or various combinations thereof. In one embodiment, the aqueous botanical extract is concentrated so as to form a concentrate of the aqueous botanical extract. Alternatively, the aqueous botanical extract is dried so as to form a solid form of the botanical extract. The aqueous botanical extract may be dried so as to contain no more than 10% of water by weight, no more than 5% of water by weight, no more than 2% of water by weight or no more than 1% of water by weight. This can be measured by moisture content determination AOAC (2000), for example. Suitable concentration and/or drying methods are well known in the art. Examples include, but are not limited to, reduced pressure evaporation, evaporation, reduced pressure distillation, distillation, oven drying, sun drying, and lyophilization (i.e. freeze-drying), spray drying, atomization or fluidized bed dryer. Concentration or drying can be performed on the aqueous botanical extract with or without a carrier or other excipients.

The extraction may be conducted in any system known in the art allowing subcritical water extraction, and include a system comprising a batch extractor or a continuous extractor.

### Botanical extract obtained from Ligustrum lucidum

In one aspect, the invention provides a botanical extract obtained from the fruit of *Ligustrum lucidum.* The botanical extract can be obtained/is obtainable from fruit of *Ligustrum lucidum* by extraction with subcritcal water where the extraction comprises the step of contacting the fruit with subcritical water at a temperature of from 40°C to 180°C and at a pressure suitable to maintain the water in a liquid state for a period of at least 5 minutes. It has been found that, under these specific extraction conditions, a novel botanical extract can be obtained and that, surprisingly, this extract has advantageous cosmetic properties. Specifically, the botanical extract is obtained/obtainable by the process described in the above section entitled "Process for obtaining a botanical extract from fruit of *Ligustrum lucidum".* The botanical extract can be obtained in the form of an aqueous botanical extract or a purified aqueous botanical extract. Details of the extraction process as described herein can be applied or incorporated into the definition of the botanical extract, aqueous botanical extract and/or purified aqueous botanical extract provided herein.

The botanical extract can be in the form of an aqueous botanical extract. In the process of the invention, the extraction with subcritical water comprises a step of contacting the fruit with subcritical water at a temperature of from 40°C to 180°C and at a pressure suitable to maintain the water in a liquid state for a period of at least 5 minutes to form an aqueous botanical extract. In other words, the extraction comprises a step of contacting the fruit with subcritical water, where the water is at a temperature of from 40°C to 180°C and at a pressure that maintains it in a liquid state, for a period of at least 5 minutes, so as to form an aqueous botanical extract. During this step, components are extracted from the fruit into the subcritical water; specifically, components of the fruit dissolve in the subcritical water to form an aqueous botanical extract. Essentially, the botanical extract is the extracted components (components which are extracted from the fruit and dissolve in the subcritical water). The aqueous botanical extract is the botanical extract in aqueous form. The aqueous botanical extract is a composition comprising the botanical extract and water. While the water is subcritical, the extracted components remain dissolved in the water and the aqueous botanical extract is a solution. After the extraction, when the water cools and is at a lower pressure, the extracted components may start to precipitate out of solution. Thus, the extracted components may or may not be completely dissolved in the aqueous botanical extract.

In the process described above, the aqueous botanical extract may be subjected to a purification step to produce a purified aqueous botanical extract. The purified aqueous extract can be a concentrate of the aqueous botanical extract or a solid form of the botanical extract, such as an amorphous solid, a crystalline or part-crystalline solid, optionally in the form of a powder.

The aqueous botanical extract or purified aqueous botanical extract comprises oleuropein, salidroside and myricetin. The aqueous botanical extract or purified aqueous botanical extract may comprise oleuropein, salidroside, myricetin and coniferyl alcohol. The aqueous botanical extract or purified aqueous botanical extract may comprise oleuropein, salidroside, myricetin and apigenin. The aqueous botanical extract or purified aqueous botanical extract may comprise oleuropein, salidroside, myricetin, coniferyl alcohol and apigenin.

The aqueous botanical extract or purified aqueous botanical extract may comprise oleuropein, salidroside, myricetin and at least one compound selected from vanillin, 3,4-dihydroxybenzoic acid, 3,5-dihydroxybenzoic acid, 4-hydroxybenzaldehyde, 4-hydroxybenzoic acid, caffeic acid, quercetin, sinapyl-alcohol, salicylic acid, kaempferol and naringenin. The aqueous botanical extract or purified aqueous botanical extract may comprise oleuropein, salidroside, myricetin, vanillin, 3,4-dihydroxybenzoic acid and/or 3,5-dihydroxybenzoic acid, 4-hydroxybenzoic acid, caffeic acid, sinapyl-alcohol, salicylic acid and kaempferol. The aqueous botanical extract or purified aqueous botanical extract may comprise oleuropein, salidroside, myricetin, vanillin, 3,4-dihydroxybenzoic acid, 3,5-dihydroxybenzoic acid, 4-hydroxybenzaldehyde, 4-hydroxybenzoic acid, caffeic acid, quercetin, sinapyl-alcohol, salicylic acid, kaempferol and naringenin.

The aqueous botanical extract or purified aqueous botanical extract may comprise oleuropein, salidroside, myricetin and at least one compound selected from vanillin, 4-hydroxybenzaldehyde, 4-hydroxybenzoic acid, quercetin, sinapyl-alcohol, salicylic acid, and naringenin. The aqueous botanical extract or purified aqueous botanical extract may comprise oleuropein, salidroside, myricetin, vanillin, 4-hydroxybenzoic acid, sinapyl-alcohol, and salicylic acid. The aqueous botanical extract or purified aqueous botanical extract may comprise oleuropein, salidroside, myricetin, vanillin, 4-hydroxybenzaldehyde, 4-hydroxybenzoic acid, quercetin, sinapyl-alcohol, salicylic acid, and naringenin.

The aqueous botanical extract or purified aqueous botanical extract may comprise oleuropein, salidroside, myricetin vanillin, luteolin-7-O-glucoside, 3,4-dihydroxybenzoic acid and/or 3,5-dihydroxybenzoic acid, 4-hydroxybenzaldehyde, 4-hydroxybenzoic acid, vanillic acid, caffeic acid, ferulic acid, abscisic acid, quercetin, eriodictyol, sinapyl-alcohol, salicylic acid, coniferaldehyde, apigenin-7-O-glucoside, cinnamic acid, coumaric acid kaempferol 3,5-dihydroxybenzaldehyde and naringenin.

The aqueous botanical extract or purified aqueous botanical extract may comprise oleuropein, salidroside, myricetin, coniferyl alcohol and at least one compound selected from vanillin, 3,4-dihydroxybenzoic acid, 3,5-dihydroxybenzoic acid, 4-hydroxybenzaldehyde, 4-hydroxybenzoic acid, caffeic acid, quercetin, sinapyl-alcohol, salicylic acid, kaempferol and naringenin. The aqueous botanical extract or purified aqueous botanical extract may comprise oleuropein, salidroside, myricetin, coniferyl alcohol, vanillin, 3,4-dihydroxybenzoic acid and/or 3,5-dihydroxybenzoic acid, 4-hydroxybenzoic acid, caffeic acid, sinapyl-alcohol, salicylic acid and kaempferol. The aqueous botanical extract or purified aqueous botanical extract may comprise oleuropein, salidroside, myricetin, coniferyl alcohol, vanillin, 3,4-dihydroxybenzoic acid, 3,5-dihydroxybenzoic acid, 4-hydroxybenzaldehyde, 4-hydroxybenzoic acid, caffeic acid, quercetin, sinapyl-alcohol, salicylic acid, kaempferol and naringenin.

The aqueous botanical extract or purified aqueous botanical extract may comprise oleuropein, salidroside, myricetin, coniferyl alcohol and at least one compound selected from vanillin, 4-hydroxybenzaldehyde, 4-hydroxybenzoic acid, quercetin, sinapyl-alcohol, salicylic acid, and naringenin. The aqueous botanical extract or purified aqueous botanical extract may comprise oleuropein, salidroside, myricetin, coniferyl alcohol, vanillin, 4-hydroxybenzoic acid, sinapyl-alcohol, and salicylic acid. The aqueous botanical extract or purified aqueous botanical extract may comprise oleuropein, salidroside, myricetin, coniferyl alcohol, vanillin, 4-hydroxybenzaldehyde, 4-hydroxybenzoic acid, quercetin, sinapyl-alcohol, salicylic acid, and naringenin.

The aqueous botanical extract or purified aqueous botanical extract may comprise oleuropein, salidroside, myricetin, coniferyl alcohol, vanillin, luteolin-7-O-glucoside, 3,4-dihydroxybenzoic acid and/or 3,5-dihydroxybenzoic acid, 4-hydroxybenzaldehyde, 4-hydroxybenzoic acid, vanillic acid, caffeic acid, ferulic acid, abscisic acid, quercetin, eriodictyol, sinapyl-alcohol, salicylic acid, coniferaldehyde, apigenin-7-O-glucoside, cinnamic acid, coumaric acid kaempferol 3,5-dihydroxybenzaldehyde and naringenin.

The aqueous botanical extract or purified aqueous botanical extract may comprise oleuropein, salidroside, myricetin, apigenin and at least one compound selected from vanillin, 3,4-dihydroxybenzoic acid, 3,5-dihydroxybenzoic acid, 4-hydroxybenzaldehyde, 4-hydroxybenzoic acid, caffeic acid, quercetin, sinapyl-alcohol, salicylic acid, kaempferol and naringenin. The aqueous botanical extract or purified aqueous botanical extract may comprise oleuropein, salidroside, myricetin, apigenin, vanillin, 3,4-dihydroxybenzoic acid and/or 3,5-dihydroxybenzoic acid, 4-hydroxybenzoic acid, caffeic acid, sinapyl-alcohol, salicylic acid and kaempferol. The aqueous botanical extract or purified aqueous botanical extract may comprise oleuropein, salidroside, myricetin, apigenin, vanillin, 3,4-dihydroxybenzoic acid, 3,5-dihydroxybenzoic acid, 4-hydroxybenzaldehyde, 4-hydroxybenzoic acid, caffeic acid, quercetin, sinapyl-alcohol, salicylic acid, kaempferol and naringenin.

The aqueous botanical extract or purified aqueous botanical extract may comprise oleuropein, salidroside, myricetin, apigenin and at least one compound selected from vanillin, 4-hydroxybenzaldehyde, 4-hydroxybenzoic acid, quercetin, sinapyl-alcohol, salicylic acid, and naringenin. The aqueous botanical extract or purified aqueous botanical extract may comprise oleuropein, salidroside, myricetin, apigenin, vanillin, 4-hydroxybenzoic acid, sinapyl-alcohol, and salicylic acid. The aqueous botanical extract or purified aqueous botanical extract may comprise oleuropein, salidroside, myricetin, apigenin, vanillin, 4-hydroxybenzaldehyde, 4-hydroxybenzoic acid, quercetin, sinapyl-alcohol, salicylic acid, and naringenin.

The aqueous botanical extract or purified aqueous botanical extract may comprise oleuropein, salidroside, myricetin, apigenin, vanillin, luteolin-7-O-glucoside, 3,4-dihydroxybenzoic acid and/or 3,5-dihydroxybenzoic acid, 4-hydroxybenzaldehyde, 4-hydroxybenzoic acid, vanillic acid, caffeic acid, ferulic acid, abscisic acid, quercetin, eriodictyol, sinapyl-alcohol, salicylic acid, coniferaldehyde, apigenin-7-O-glucoside, cinnamic acid, coumaric acid kaempferol 3,5-dihydroxybenzaldehyde and naringenin.

The aqueous botanical extract or purified aqueous botanical extract may comprise oleuropein, salidroside, myricetin, coniferyl alcohol, apigenin and at least one compound selected from vanillin, 3,4-dihydroxybenzoic acid, 3,5-dihydroxybenzoic acid, 4-hydroxybenzaldehyde, 4-hydroxybenzoic acid, caffeic acid, quercetin, sinapyl-alcohol, salicylic acid, kaempferol and naringenin. The aqueous botanical extract or purified aqueous botanical extract may comprise oleuropein, salidroside, myricetin, coniferyl alcohol, apigenin, vanillin, 3,4-dihydroxybenzoic acid and/or 3,5-dihydroxybenzoic acid, 4-hydroxybenzoic acid, caffeic acid, sinapyl-alcohol, salicylic acid and kaempferol. The aqueous botanical extract or purified aqueous botanical extract may comprise oleuropein, salidroside, myricetin, coniferyl alcohol, apigenin, vanillin, 3,4-dihydroxybenzoic acid, 3,5-dihydroxybenzoic acid, 4-hydroxybenzaldehyde, 4-hydroxybenzoic acid, caffeic acid, quercetin, sinapyl-alcohol, salicylic acid, kaempferol and naringenin.

The aqueous botanical extract or purified aqueous botanical extract may comprise oleuropein, salidroside, myricetin, coniferyl alcohol, apigenin and at least one compound selected from vanillin, 4-hydroxybenzaldehyde, 4-hydroxybenzoic acid, quercetin, sinapyl-alcohol, salicylic acid, and naringenin. The aqueous botanical extract or purified aqueous botanical extract may comprise oleuropein, salidroside, myricetin, coniferyl alcohol, apigenin, vanillin, 3,4-dihydroxybenzoic acid and/or 3,5-dihydroxybenzoic acid, 4-hydroxybenzoic acid, caffeic acid, sinapyl-alcohol, salicylic acid and kaempferol. The aqueous botanical extract or purified aqueous botanical extract may comprise oleuropein, salidroside, myricetin, coniferyl alcohol, apigenin, vanillin, 3,4-dihydroxybenzoic acid, 3,5-dihydroxybenzoic acid, 4-hydroxybenzaldehyde, 4-hydroxybenzoic acid, caffeic acid, quercetin, sinapyl-alcohol, salicylic acid, kaempferol and naringenin.

The aqueous botanical extract or purified aqueous botanical extract may comprise oleuropein, salidroside, myricetin, coniferyl alcohol, apigenin, vanillin, luteolin-7-O-glucoside, 3,4-dihydroxybenzoic acid and/or 3,5-dihydroxybenzoic acid, 4-hydroxybenzaldehyde, 4-hydroxybenzoic acid, vanillic acid, caffeic acid, ferulic acid, abscisic acid, quercetin, eriodictyol, sinapyl-alcohol, salicylic acid, coniferaldehyde, apigenin-7-O-glucoside, cinnamic acid, coumaric acid kaempferol 3,5-dihydroxybenzaldehyde and naringenin.

### Stock Solutions

As described above, the botanical extract obtained from the fruit of *Ligustrum lucidum* by extraction with subcritcal water can be in aqueous form, i.e. in the form of an aqueous botanical extract. The extracted components from the fruit may or may not be fully dissolved in the aqueous botanical extract. Advantageously, the aqueous botanical extract can be mixed with a water-miscible organic solvent so as to keep the extracted components in solution. The process for obtaining the botanical extract of the invention can include a step comprising mixing an aqueous botanical extract as defined herein with a water-miscible organic solvent. The resultant composition comprises an aqueous botanical extract and a water-miscible organic solvent. Water can be added to this composition to obtain the required concentration of water-miscible solvent, for example. The process for obtaining the botanical extract of the invention can include a step comprising mixing a purified aqueous botanical extract as defined herein with a water-miscible organic solvent and, optionally, water. The resultant composition comprises a purified aqueous botanical extract with a water-miscible organic solvent and, optionally, water. The purified aqueous botanical extract can be a concentrate of the aqueous botanical extract or a solid form of the botanical extract. These compositions are also referred to herein as "stock solutions".

The water-miscible organic solvent is preferably a cosmetically acceptable organic solvent and can be a polyol. Suitable polyols included glycols which are organic compounds containing two alcohol functions (-OH groups), such as: C2-C10 aliphatic hydrocarbyl diols or the triol, glycerin. C2-C10 aliphatic hydrocarbyl diols include C2-C10 or C2-C8 alkanediols in each isomeric form and which can be substituted or unsubstituted. When substituted, the alkane diols can be substituted with one or more substituents independently selected from halo, hydroxyl, ester, nitro, cyano, haloalkyl, sulfonyl and carbonyl groups, for example. The water-miscible organic solvent can be chosen from: 1,2-propanediol, ethylene glycol, diethylene glycol, propylene glycol, dipropylene glycol, butylene glycol, pentylene glycol, glycerol or caprylyl glycol and mixtures thereof. Particularly, the polyol is glycerol (also referred to herein as glycerine or glycerin).

The composition comprising the aqueous botanical extract and the polyol and optional (additional) water can have a concentration of polyol is at least 50%, or from 50% to 90%, or from 50 to 80 %, or from 55 to 80 % by weight based on the total weight of the compositon. Typically the balance is made up with the aqueous botanical extract and water. For example, the composition can comprise up to 50 % water by weight. Preferably, the polyol is glycerin. The composition comprising the purified botanical extract and the polyol and water can have a concentration of polyol is at least 50%, or from 50% to 90%, or from 50 to 80 %, or from 55 to 80 % by weight based on the total weight of the compositon. Typically the balance is made up with the purified botanical extract and water. For example, the composition can comprise up to 50 % water by weight. Preferably, the polyol is glycerin.

These compositions may comprise from about 10 to 100 ppm, or from about 20 to 80 ppm, or from 30 to 60 ppm of polyphenols. In particular the compositions comprising the aqueous botanical extract and the polyol and optional (additional) water may comprise from about 10 to 100 ppm, or from about 20 to 80 ppm, or from 30 to 60 ppm of polyphenols. Polyphenols are organic compounds characterized by multiples of phenol units and include flavonoids, phenolic acids, coumarins, and stilbenes.

These compositions comprise either the aqueous botanical extract or the purified aqueous botanical extract and thus will comprise the components of the aqueous botanical extract or the purified aqueous botanical extract as described above.

These compositions may comprise from about 1 to about 40 ppm oleuropein, from about 1 to about 40 ppm salidroside and from about 0.1 to about 3 ppm myricetin (3,5,7-trihydroxy-2-(3,4,5-trihydroxyphenyl)chromen-4-one). Oleuropein can be present in an amount of from about 10 to 30 ppm, or from about 12 to 25 ppm, or from about 20 to 30 ppm. Salidroside can be present in an amount of from about 1 to about 22 ppm or from about 10 to 20 ppm. Myricetin can be present in an amount of from about 0.2 to about 2 ppm, or from about 0.5 to about 1.5 ppm.

These compositions may additionally comprise from about 1 or 2 to about 25 ppm coniferyl-alcohol (4-[(E)-3-hydroxyprop-1-enyl]-2-methoxyphenol) and/or from about 0.01 to about 5 ppm apigenin. Coniferyl-alcohol can be present in an amount of from about 2 to about 15 ppm, or from about 4 to about 9 ppm. Apigenin can be present in an amount of from about 0.05 to about 3 ppm or from about 1 to about 2.5 ppm.

When present in these compositions, naringenin may be present in an amount of from about 0.001 to about 0.01 ppm naringenin, or from about 0.001 to about 0.02 ppm, or from about 0.001 to about 0.005 ppm.

When present in these compositions, vanillin (4-Hydroxy-3-methoxybenzaldehyde) may be present in an amount of from about 0.05 to about 2 ppm or from about 0.1 to about 1 ppm, or from about 0.2 to about 0.5 ppm.

When present in these compositions, 4-hydroxybenzaldehyde may be present in an amount of from about 0.01 to about 0.5 ppm, or from about 0.02 to about 0.2 ppm, or from about 0.04 to about 0.1 ppm.

When present in these compositions, 4-hydroxybenzoic acid may be present in an amount of from about 0.1 to about 1 ppm or from about 0.05 to about 0.5 ppm, or from about 0.1 to about 0.3 ppm.

When present in these compositions, quercetin (2-(3,4-dihydroxyphenyl)-3,5,7-trihydroxychromen-4-one) may be present in an amount of from about 0.05 to about 0.3 ppm, or from about 0.01 to about 0.15 ppm, or from about 0.03 to about 0.07 ppm.

When present in these compositions, (4-[(E)-3-hydroxyprop-1-enyl]-2,6-dimethoxyphenol) may be present in an amount of from about 0.1 to about 3 ppm or from about 0.2 to about 2 ppm, or from about 0.4 to about 1 ppm.

### Cosmetic Compositions

The botanical extract of the invention can be administered by any means that causes contact between the botanical extract and the skin of a subject's body and can be in the form of a composition that contains the botanical extract. Preferably, the subject is a mammal, more preferably a human.

In another aspect, the invention provides a cosmetic composition comprising an aqueous botanical extract, a purified aqueous botanical extract, or a stock solution, all as defined herein, together with at least one cosmetically acceptable excipient or adjuvant. These compositions can be prepared by conventional means known to persons skilled in the art *["*Harry's Cosmeticology", Seventh edition, (1982), Wilkinson J.B., Moore R.J., ed. Longman House, Essex, GB*].*

The cosmetic composition contains a cosmetically effective amount of the botanical extract of the invention which should be administered, as well as their dosage, will depend on numerous factors, including age, state of the patient, the nature or severity of the condition, disorder or disease to be treated and/or cared for, the route and frequency of administration and of the particular nature of the compounds to be used.

The term "cosmetically effective amount" is understood to mean a non-toxic but sufficient amount of extract of the invention to provide the desired effect. The extracts or stock solutions of the invention are used in cosmetic compositions of this invention at cosmetically effective concentrations to achieve the desired effect; for example in amounts with respect to the total weight of the composition of: from 0.00000001% (in weight) to 20% (in weight); from 0.000001% (in weight) to 15% (in weight), from 0.00001% (in weight) to 10% (in weight); or from 0.0001% (in weight) to 5% (in weight); or from 0.1 to 4% (in weight); or from 1 to 3% (In weight). For example, the aqueous botanical extract or the stock solution of the invention can be present in the cosmetic composition in an amount of from 0.1 to 4% (in weight); or from 1 to 3% (In weight); or from 1.5 to 2.5 % (in weight); or 2% (in weight).

The extract, stock solutions and compositions according to the invention can also be incorporated into fabrics, non-woven fabrics and medical devices which are in direct contact with the skin, thus releasing the compounds of the invention whether by biodegradation of the binding system to the fabric, non-woven fabric or medical device, or by friction between them and the body, due to bodily moisture, the skin's pH or body temperature. Furthermore, the compounds of the invention can be incorporated into the fabrics and non-woven fabrics used to make garments that are in direct contact with the body.

Examples of fabrics, non-woven fabrics, garments, medical devices and means for immobilizing the compounds to them, among which are the delivery systems and/or the sustained release systems described above, can be found in literature and are known in the prior art *[*Schaab C.K. (1986) HAPPI May 1986*;* Nelson G., "Application of microencapsulation in textiles", (2002), Int. J. Pharm., 242(1-2), 55-62; *"*Biofunctional Textiles and the Skin" (2006) Curr. Probl. Dermatol. v.33, Hipler U.C. and Elsner P., eds. S. Karger AG, Basel, Switzerl*and;* Malcolm R.K. et al., "Controlled release of a model antibacterial drug from a novel self-lubricating silicone biomaterial", (2004), J. Cont. Release, 97(2), 313-320*].* The preferred fabrics, non-woven fabrics, garments and medical devices are bandages, gauzes, t-shirts, socks, tights, underwear, girdles, gloves, diapers, sanitary napkins, dressings, bedspreads, wipes, adhesive patches, non-adhesive patches, occlusive patches, microelectric patches and/or face masks.

The invention extends to fabrics, non-woven fabrics and medical devices which have the extracts incorporated, as described above.

The cosmetic compositions of the invention can be compositions for topical or transdermal application which optionally include cosmetically or pharmaceutically acceptable excipients necessary for formulating the desired administration form.

Compositions for topical or transdermal application can be produced in any solid, liquid or semisolid formulation, such as and not restricted to, creams, multiple emulsions such as and not restricted to, oil and/or silicone in water emulsions, water-in-oil and/or silicone emulsions, water/oil/water or water/silicone/water type emulsions and oil/water/oil or silicone/water/silicone type emulsions, anhydrous compositions, aqueous dispersions, oils, milks, balsams, foams, lotions, gels, cream gels, hydroalcoholic solutions, hydroglycolic solutions, hydrogels, liniments, sera, soaps, shampoos, conditioners, serums, polysaccharide films, ointments, mousses, pomades, powders, bars, pencils and sprays or aerosols (sprays), including leave-on and rinse-off formulations. These topical or transdermal application formulations can be incorporated using techniques known by the person skilled in the art into different types of solid accessories for example and not restricted to, bandages, gauzes, t-shirts, socks, tights, underwear, girdles, gloves, diapers, sanitary napkins, dressings, bedspreads, wipes, adhesive patches, non-adhesive patches, occlusive patches, microelectric patches or face masks, or they can be incorporated into different make-up products such as make-up foundation, such as fluid foundations and compact foundations, make-up removal lotions, make-up removal milks, under-eye concealers, eye shadows, lipsticks, lip protectors, lip gloss and powders among others.

In one embodiment the cosmetic composition of the invention is in a form chosen from creams, multiple emulsions, solutions, liquid crystals, anhydrous compositions, aqueous dispersions, oils, milks, balsams, foams, lotions, gels, cream gels, hydroalcoholic solutions, hydroglycolic solutions, hydrogels, liniments, soaps, shampoos, conditioners, serums, polysaccharide films, ointments, mousses, pomades, powders, bars, pencils, sprays or aerosols.

The cosmetic compositions of the invention may include agents which increase the percutaneous absorption of the compounds of the invention, for example and not restricted to, dimethylsulfoxide, dimethylacetamide, dimethylformamide, surfactants, azone (1-dodecylazacycloheptane-2-one), alcohol, urea, ethoxydiglycol, acetone, propylene glycol or polyethylene glycol, among others. Furthermore, the cosmetic compositions of this invention can be applied to local areas to be treated by means of iontophoresis, sonophoresis, electroporation, microelectric patches, mechanical pressure, osmotic pressure gradient, occlusive cure, microinjections or needle-free injections by means of pressure, such as injections by oxygen pressure, or any combination thereof, to achieve a greater penetration of the peptide of the invention. The application area will be determined by the nature of the condition, disorder and/or disease to be treated and/or cared for.

Cosmetic compositions according to the invention can also be administered, as well as by topical or transdermal route, by any other appropriate route, such as oral or parenteral route, for which they will include the pharmaceutically acceptable excipients necessary for the formulation of the desired administration form. In the context of this invention, the term "parenteral" includes nasal, auricular, ophthalmic, rectal, urethral, vaginal, subcutaneous, intradermal route, intravascular injections, such as intravenous, intramuscular, intraocular, intravitreous, intracorneal, intraspinal, intramedullary, intracranial, intracervical, intracerebral, intrameningeal, intraarticular, intrahepatic, intrathoracic, intratracheal, intrathecal and intraperitoneal, and any another similar injection or infusion technique. A person skilled in the art knows the different means by which the cosmetic compositions which contain the compounds of the invention can be administered.

Alternatively, the extracts and compositions of the invention can be used in different types of formulations for oral administration, preferably in the form of oral cosmetics or drugs, such as and not restricted to, capsules, including gelatin capsules, soft capsules, hard capsules, tablets, including sugar coated tablets, tablets, pills, powders, granules, chewing gum, solutions, suspensions, emulsions, syrups, elixirs, polysaccharide films, jellies or gelatins, and any other form known by the person skilled in the art. In a particular embodiment, the compounds of the invention can be incorporated into any form of functional food or fortified food, such as and not restricted to, dietary bars or compact or non-compact powders. These powders can be dissolved in water, soda, dairy products, soy derivatives or can be incorporated into dietary bars. The extract and compositions of this invention can be formulated with common excipients and adjuvants for oral compositions or food supplements, for example and not restricted to, fat components, aqueous components, humectants, preservatives, texturizing agents, flavors, aromas, antioxidants and colorants common in the food industry.

Among the cosmetically or pharmaceutically acceptable adjuvants contained in the cosmetic compositions described in this invention are additional ingredients commonly used in cosmetic or pharmaceutical compositions, for example and not restricted to anti-wrinkle agents, botox-like agents and/or anti-aging agents; (ii) firming agents, skin elasticity agents and/or restructuring agents; moisturizing agents; (iv) anti-photoaging agents, and/or blue-light protector agents; DNA protecting agents, DNA repair agents, and/or stem cell protecting agents; free radical scavengers and/or anti-glycation agents, detoxifying agents, antioxidant and/or anti-pollution agents; antiperspirant agents; melanin synthesis stimulating or inhibiting agents; whitening or depigmenting agents; propigmenting agents; self-tanning agents; lipolytic agents or agents stimulating lipolysis, adipogenic agents, etc. Additional examples can be found in CTFA International Cosmetic Ingredient Dictionary & Handbook, 12th Edition (2008*).*

In one embodiment, the invention provides a cosmetic composition comprising the extract of the invention (e.g. in the form of the aqueous botanical extract, purified aqueous botanical extract or stock solution) and a cosmetically effective amount of an ingredient selected from the group consisting of: (i) anti-wrinkle-agent, botox-like agent and/or anti-aging agent; (ii) firming agent, skin elasticity agent and/or restructuring agent; (iii) moisturizing agent; (iv) anti-photoaging agent, and/or blue-light protector agent; (v) DNA protecting agent, DNA repair agent, and/or stem cell protecting agent; (vi) free radical scavengers and/or anti-glycation agent, detoxifying agent, antioxidant and/or anti-pollution agents; and/or combinations thereof.

In a particular embodiment, the anti-wrinkle agent, botox-like agent and/or anti-aging agent is selected from thee group consisting of Oxygestkin^{®} [INCI: *Tropaeolum majus* Flower/Leaf/Stem Extract] or Eclaline^{™}. [INCI: Hydrolyzed Lupine Protein Octenylsuccinate] from SILAB; Matrixyl^{®} [INCI: Palmitoyl Pentapeptide-4], Matrixyl^{®} 3000^{®} [INCI: Palmitoyl Tetrapeptide-7, Palmitoyl Oligopeptide], Matrixyl^{®} Synthe'6 [INCI: Glycerin, Water, Hydroxypropyl Cyclodextrin, Palmitoyl Tripeptide-38], Matrixyl^{®} Morphomics^{™} [INCI: Pentylene Glycol, Caprylyl Glycol], Essenskin^{™} [INCI: calcium hydroxymethionine], Renovage [INCI: Teprenone], Dermaxyl^{®} [INCI: Palmitoyl Oligopeptide], Calmosensine [INCI: Butylene Glycol, Acetyl Dipeptide-1 Cetyl Ester], Volulip [INCI: Cetearyl Ethylhexanoate, Sorbitan Isostearate, Portulaca Pilosa Extract, Sucrose Cocoate, Palmitoyl Tripeptide-38], Subliskin [INCI: Sinorhizobium Meliloti Ferment, Cetyl Hydroxyethyl Cellulose, Lecithin], Biopeptide CL [INCI: Palmitoyl Oligopeptide], Biopeptide EL [INCI: Palmitoyl Oligopeptide], Rigin [INCI: Palmitoyl Tetrapeptide-3], Biobustyl [INCI: Glyceryl Polymethacrylate, Rahnella/Soy Protein Ferment, Palmitoyl Oligopeptide], Dynalift [INCI: Sodium Polystyrene Sulfonate, Sorghum Bicolor Stalk Juice, Glycerin], Idealift [INCI: Acetyl Dipeptide-1 Cetyl Ester], Siegesbeckia [INCI: Siegesbeckia Orientales Extract], Ovaliss [INCI: Coco-glucoside, Caprylyl Glycol, Alcohol, Glaucine], Juvinity^{™} [INCI: Geranylgeranyisopropanol], Prolevis [INCI: Hydrolyzed Vegetable Protein], Idealift^{™} [INCI: Hydroxyethylcellulose, Acetyl Dipeptide-1 cetyl ester], Beautifeye^{™} [INCI: Albizia Julibrissin Bark Extract, Darutoside], Chromocare^{™} [INCI: Sigesbeckia Orientalis Extract, Rabdosia Rubescens Extract] or Resistem^{™} [INCI proposed: Globularia Cordifolia Ferment] marketed by Sederma/Croda. Vialox^{®} [INCI: Pentapeptide-3], Syn^{®}-Ake^{®} [INCI: Dipeptide Diaminobutyroyl Benzylamide Diacetate], Syn^{®}-Coll [INCI: Palmitoyl Tripeptide-5], Phytaluronate [INCI: Ceratonia Siliqua (Carob) Gum], Preregen^{®} [INCI: Glycine soja (Soybean) Protein, Oxido Reductases], Pepha-Nutrix [INCI: Natural Nutrition Factors], Pepha-Tight [INCI: Algae Extract, Pullulan], Pentacare-NA [INCI: Hydrolyzed Wheat Gluten, Ceratonia Siliqua Gum], Syn^{®}-Tacks [INCI: Glycerin, Palmitoyl Dipeptide-5 Diaminobutyloyl Hydroxythreonine, Palmitoyl Dipeptide-6 Diaminohydroxybutyrate], BeauActive MTP [INCI: Hydrolyzed milk protein], Syn^{®}-TC [INCI: Tetradecyl Aminobutyroylvalylaminobutyric Urea Trifluoroacetat, Palmitoyl Tripeptide-5, Palmitoyl Dipeptide-5 Diaminobutyroyl Hydroxythreonine], Syn^{®}-Hycan [INCI: Tetradecyl Aminobutyroylvalylaminobutyric Urea Trifluoroacetate], Syn^{®}-Glycan [INCI: Tetradecyl Aminobutyroylvalyl-aminobutyric Urea Trifluoroacetate], Regu-Age [INCI: Hydrolyzed Rice Bran Protein, Oxido Reductases, Glycine Soja Protein], Pepha-Timp [INCI: Human oligopeptide-20], Pepha-Age [INCI: Dunaliella Salina Extract], Colhibin [INCI: Hydrolyzed Rice Protein], Elhibin [INCI: Glycine Soja Protein, Disodium cocoamphodiacetate] or All-Q^{™} Plus [INCI: Ubiquinone, Tocopheryl Acetate] marketed by Pentapharm/DSM; Myoxinol^{™} [INCI: Hydrolyzed Hibiscus esculentus Extract], Myoxinol^{™} LS 9736 [INCI: Hydrolyzed Hibiscus esculentus Extract, Dextrin], Syniorage^{™} [INCI: Acetyl Tetrapeptide-11], Dermican^{™} [INCI: Acetyl Tetrapeptide-9], DN-AGE^{®} LS [INCI: Cassia alata leaf Extract], Hyalufix GL [INCI: Alpinia Galanga Leaf Extract], Neurobiox [INCI: Achillea Millefolium Extract,], Deliner [INCI: Zea Mays (Corn) Kernel Extract], Lys'lastine V [INCI: Peucedanum Graveolens (Dill) Extract], Extracellium [INCI: Hydrolyzed Potato Protein], Proteasyl TP LS 8657 [INCI: Pisum Sativum Extract], Flavagrum PEG [INCI: PEG-6 Isostearate, Hesperetin Laurate], Micromerol [INCI: Pyrus Malus Fruit Extract], Extracellium [INCI: Hydrolyzed Potato Protein], Marine Filling Spheres [INCI: Pentaerythrityl Tetraisostearate, Silica Dimethyl Silylate, Sodium Chondroitin Sulfate, Atelocollagen], Triactigen [INCI: Mannitol, Cyclodextrin, Yeast Extract, Disodium Succinate], Eterniskin [INCI: Grifola Frondosa Fruiting Body Extract, Maltodextrin], Ascotide [INCI: Ascorbyl Phosphate Succinoyl Pentapeptide-12], Hyalurosmooth [INCI: Cassia Angustifolia Seed Polysaccharide], Indinyl CA [INCI: Cassia Angustifolia Seed Polysaccharide], Arganyl [INCI: Argania Spinosa Leaf Extract], Sphingoceryl Veg [INCI: Phyto-ceramides], Vit-A-Like [INCI: Vigna Acontifolia Seed Extract], Peptiskin [INCI: Arginine/Lysine polypeptide], Prodejine [INCI: Mannitol, Cyclodextrin, Yeast Extract, Disodium Succinate], Aqu'activ [INCI: Behenyl Alcohol, Glyceryl Oleate, Cocamide MIPA, Calcium Citrate], Elestan [INCI: Glycerin, Manilkara Leaf Extract], Hibiscin HP [INCI: Hibiscus Esculentus Seed Extract], Collalift^{®}18 [INCI: Khaya Senegalensis Bark], Collrepair^{™} DG [INCI: Hexylene Glycol, Niacin] or Litchiderm [INCI: Litchi Chinensis Pericarp Extract] marketed by Laboratoires Sérobiologiques/Cognis/BASF; Argireline^{®} [INCI: Acetyl Hexapeptide-8], SNAP-7 [INCI: Acetyl Heptapeptide-4], SNAP-8 [INCI: Acetyl Octapeptide-3], Leuphasyl^{®} [INCI: Pentapeptide-18], Inyline^{®} [INCI: Acetyl Hexapeptide-30], Aldenine^{®} [INCI: Hydrolized Wheat Protein, Hydrolized Soy Protein, Tripeptide-1], Preventhelia^{®} [INCI: Diaminopropionoyl Tripeptide-33], Decorinyl^{®} [INCI: Tripeptide-10 Citrulline], Decorinol^{®} [INCI: Tripeptide-9 Citrulline], Trylagen^{®} [INCI: Pseudoalteromonas Ferment Extract, Hydrolyzed Wheat Protein, Hydrolyzed Soy Protein, Tripeptide-10 Citrulline, Tripeptide-1], Eyeseryl^{®} [INCI: Acetyl Tetrapeptide-5], Peptide AC29 [INCI: Acetyl Tripeptide-30 Citrulline], Relistase^{®} [INCI: Acetylarginyltriptophyl Diphenylglycine], Thermostressine^{®} [INCI: Acetyl Tetrapeptide-22], Lipochroman^{™} [INCI: Dimethylmethoxy Chromanol], Chromabright^{®} [INCI: Dimethylmethoxy Chromanyl Palmitate], Antarcticine^{®} [INCI: Pseudoalteromonas Ferment Extract], dGlyage^{®} [INCI: Lysine HCl, Lecithin, Tripeptide-9 Citrulline], Vilastene^{™} [INCI: Lysine HCl, Lecithin, Tripeptide-10 Citrulline], Hyadisine^{®} [INCI: Pseudoalteromonas Ferment Extract], Hyanify^{™} [INCI: Saccharide Isomerate], Diffuporine^{®} [INCI: Acetyl Hexapeptide-37], Silusyne^{®} [INCI: Soybean (Glycine Soja) Oil, Sorbitan Sesquioleate, Isohexadecane, Sodium Hyaluronate, Lauryldimonium Hydroxypropyl Hydrolized Soy Protein, Acetyl Hexapeptide-39], Adifyline^{®} [INCI: Acetyl Hexapeptide-38], Delisens^{™} [INCI: Acetyl Hexapeptide-46], Telangyn^{™} [INCI: Acetyl Tetrapeptide-40], Reproage^{™} peptide [INCI: Acetyl Hexapeptide-8], Cellynkage^{™} marine ingredient [INCI: Saccharide Isomerate], Eyedeline^{™} marine ingredient [INCI: Plankton Extract], Uplevity^{™} [INCI: Acetyl Tetrapeptide-2], Seacode^{™} marine ingredient [INCI: Pseudoalteromonas Ferment Extract] or Serilesine^{®} peptide solution [INCI: Hexapeptide-10]; Argireline^{®} Amplified [INCI: Acetyl Hexapeptide-8], Dawnergy^{™} [INCI: Nonapeptide-1] marketed by Lipotec/Lubrizol; Sirtalice^{™} [INCI: Bacillus Ferment], Epitensive^{™} [INCI: Nicotiana Benthamiana Hexapeptide-40 SH-Oligopeptide-1], Scelleye^{™} [INCI: Nicotiana Benthamiana SH-Oligopeptide-2], Seadermium [INCI: Aqua, Glycerin, Bacillus Ferment], Pauseile [INCI: Aqua, Glycerin, Bacillus Ferment] or Neoclair pro [INCI: Aqua, Glycerin, Caprylyl Glycol, Acetyl Tetrapeptide-2] marketed by Lipotrue; Collaxyl^{®} IS [INCI: Hexapeptide-9], Laminixyl IS^{™} [INCI: Heptapeptide], Orsirtine^{™} GL [INCI: Oryza sativa (Rice) Extract], D'Orientine^{™} IS [INCI: Phoenix dactylifera (Date) Seed Extract], Phytoquintescine^{™} [INCI: Einkorn (Triticum monococcum) Extract], Quintescine^{™} IS [INCI: Dipeptide-4], Peptide Vinci 01 [INCI: Penta-decapeptide-1], Peptide Vinci 02^{™} [INCI: Hexapeptide-3], Aquarize IS^{™} [INCI: Hydrolyzed Rice Extract], Lanablue [INCI: Algae extract], Ederline^{™} [INCI: Pyrus Malus (Apple) Seed Extract], Dynachondrine^{™} ISR [INCl:Hydrolized Soy Protein], Prolixir S20^{™} [INCI: Dimer Tripeptide-43], Phytocohesine^{™} PSP [INCI: Sodium Beta-Sitosteryl Sulfate, Beta-Sitosterol], Perenityl^{™} IS [INCI: Pyrus Communis (Pear) Seed Extract], Caspaline 14^{™} [INCl:Hexapeptide-42], Peptide Q10^{™} [INCI:Pentapeptide-34 Trifluoroacetate], Survixyl IS^{™} [INCI: Pentapeptide-31], ChroNOgen^{™} [INCI: Tetrapeptide-26], Elixiance [INCI: Schinus Molle Extract], Harmoniance^{™} [INCI: Nelumbo Nucifera Flower Extract], Serenityl [INCI: Marsdenia Condurango Bark Extract], Natriance Wrinkle-less [INCI: Hydrolyzed Corn Protein], Phytoneomatrix [INCI: Hydrolyzed Soybean Extract], Prolixir ICE [INCI: Hydrolyzed Rice Protein], PhytoRNx Baobab^{™} [INCI: Hydrolyzed Adansonia Digitata Extract], Natriance Renovate Extract [INCI: Hydrolyzed Linseed Extract], Natriance Self-Hydrate Extract [INCI: Pisum Sativum Extract], Actopontine YST [INCI: Hydrolyzed Yeast Protein] or Telosense^{™} [proposed INCI: Hydrolized Soy Protein, Hydrolized Yeast Protein] marketed by Vincience/ISP/Ashland; BONT-L-Peptide [INCI: Palmitoyl Hexapeptide-19], TIMP Peptide [INCI: Acetylhexapeptide-20], ECM Moduline [INCI: Palmitoyl Tripeptide-28], Renaissance [INCI: Hydrolyzed Wheat Protein, Palmitoyl Decapeptide-21, Decapeptide-22, Oligopeptide-78, Zinc Palmitoyl Nonapeptide-14] or X50 Antiaging [INCI: Lactic Acid/glycolic Acid Copolymer, Polyvinyl Alcohol, Copper Palmitoyl Heptapeptide-14, Heptapeptide-15 Palmitate] marketed by Infinitec Activos; EquiStat [INCI: Pyrus malus Fruit Extract, Glycine soja Seed Extract], Juvenesce [INCI: Ethoxydiglicol and Caprylic Triglycerid, Retinol, Ursolic Acid, Phytonadione, Ilomastat], Ursolisome [INCI: Lecithin, Ursolic Acid, Atelocollagen, Xanthan Gum, Sodium chondroitin sulfate], Basaline [INCI: Hydrolyzed Malt Extract], Phytokine [INCI: Hydrolyzed Soy Protein], marketed by Coletica/Engelhard/BASF; Ameliox [INCI: Carnosine, Tocopherol, Silybum marianum Fruit Extract] or PhytoCellTec Malus Domestica [INCI: Malus domestica Fruit Cell Culture], Lipobelle Soyaglicane [INCI: Soy Isoflavones], RoyalEpigen P5 [INCI: Butyrospermum Parkii BUtter, Hydrogenated Lecithin, Maltodextrin, Pentapeptide-48, Phenethyl Alcohol, Ethylhexylglycerin, Glycerin, Aqua] or DermCom [INCI: Crocus Chrysanthus Bulb Extract, Acacia Senegal Gum, Aqua/Water] marketed by Mibelle Biochemistr;ActiMatrix [INCI: Peptide based mushroom Extract], Peptamide 6 [INCI: Hexapeptide-11] marketed by Active Organics/Arch; and combinations thereof.

In another embodiment, the firming agent, skin elasticity agent and/or restructuring agent is selected, from the group consisting of Argassential [INCI: C10-16 Alkyl Glucoside, Dicaprylyl Ether, Glycerin] or Replexium BC [INCI: Dimethyl Isosorbide, Polysorbate 20, Aqua, Acetyl Tetrapeptide-11, Acetyl Tetrapeptide-9] marketed by BASF; Prolevis [INCI: Hydrolyzed Vegetable Protein] or Poretect [INCI: Caprylic/capric Triglyceride, Sorbitan Trioleate, Apium Graveolens Seed Extract, Linum Usitatissimum Seed Extract] marketed by Sederma/Croda; Actifirm Ultra Advanced botanical ingredient [INCI: Centella Asiatica Extract, Rosmarinus Officinalis Leaf Extract, Dipropylene Glycol, Alcohol, Echinacea Angustifolia Leaf Extract] or Actifcol Advanced botanical ingredient [INCI: Aqua, Glycerin, Sodium Citrate, Lentinus Edodes Extract, Potassium Sorbate, Sodium Benzoate, Phytic Acid] marketed by Lipotec/Lubrizol; Densorphin^{™} [INCI: Vitex Agnus Castus Extract, Aqua, Maltodextrin] or PhytoCellTec^{™} nunatak^{®} [INCI: Isomalt, Aqua, Saponaria Pumila Callus Culture Extract, Lecithin] marketed by Mibelle; and combinations thereof.

In another embodiment, the moisturizing agent is selected from the group consisting of qua Shuttle [INCI: Sorbitol, Laminaria Digitata Extract, Diatomaceous Earth] marketed by Infinitec; Aqua-Osmoline^{™} [INCI: Ceratonia Siliqua (Carob) Seed Extract] marketed by Vincience/ISP/Ashland; Hydralphatine^{™} Asia [INCI: Hydrogenated Starch Hydrolysate, Panthenol, Bambusa Vulgaris Shoot Extract, Nelumbo Nucifera Flower Extract, Nymphaea Alba Root Extract] or Hydraporine^{™} [INCI: Betaine, Hydrogenated Lecithin, Honey, Pectin] marketed by Lucas Meyer Cosmetics/Unipex; PatcH2O^{™} [INCI: Trehalose, Urea, Serine, Glyceryl Polyacrylate, Algin, Sodium Hyaluronate, Pullulan], Aqu'activ^{™} [INCI: Behenyl Alcohol, Glyceryl Oleate, Cocamide MIPA], Irwinol^{®} [INCI: Octyldodecanol, Irvingia Gabonensis Kernel Butter, Hydrogenated Coco-Glycerides], Lipodermol^{®}) [INCI: Octyldodecanol, Arachidyl Propionate, Tocopheryl Acetate, Retinyl Palmitate, Ethyl Linoleate, Ethyl Linolenate] or Seanamin^{®} SU [INCI: Sorbitol, Algae Extract, Chrondrus Crispus (Carrageenan), Fucus Vesiculosus Extract, Algin] marketed by L. Serobiologiques/Cognis/BASF; Snow Algae Powder [INCI: Coenochloris Signiensis Extract] marketed by Mibelle; Hyasol BT [INCI: Sodium Hyaluronate], Syn-Up^{™} [INCI: Benzylsulfonyl D-Seryl Homophenylalanine Amidinobenzamide Acetate] or Pentavitin^{®} [INCI: Saccharide Isomerate] marketed by Pentapharm/DSM; Aqualance^{™} [INCI: Erythritol, Homarine HCl], Hydraprotectol^{™} [INCI: Glyceryl Polymethacrylate, Aleuritic Acid, Yeast Extract (Faex), Glycoprotein], Moist 24^{™} [INCI: Imperata Cylindrica Root Extract], Optim Hyal^{™} [INCI: Hydrolyzed Yeast Extract, Cetyl Hydroxyethylcellulose, Polyglucuronic Acid], Osmocide^{®} 4 [INCI: Glycerin, Acrylates/C10-30 Alkyl Acrylate Crosspolymer] or Revidrate^{™} [INCI: Ethylhexyl Palmitate, Sorbitan Oleate, Sorbitan Laureate, Myristyl Malate Phosphonic Acid] marketed by Sederma/Croda; Xpertmoist^{®} molecular film [INCI: Glycerin, Pseudoalteromonas Ferment Extract, Xanthan Gum, Proline, Alanine, Serine, Ethylhexylglycerin, Caprylyl Glycol] or Actizyme GL advanced botanical ingredient [INCI: Glycerin, Mucor miehei extract, Aqua, Sodium Citrate, Potassium Sorbate, Sodium Benzoate, Phytic Acid] marketed by Lipotec/Lubrizol; and combinations thereof.

In another embodiment, the anti-photoaging agent, and/or blue-light protector agent is selected from the group consisting of Algaktiv Genofix CPD [INCI: Plankton Extract, Aqua, Lecithin] marketed by Greenaltech; Blumilight^{™} Biofunctional [INCI proposed: Water/Aqua (and) Butylene Glycol (and) Theobroma Cacao (Cocoa) Seed Extract] marketed by Ashland; Lys'Sun [INCI: Hamamelis Virginiana Leaf Extract, Aqua, Pentylene Glycol, Caprylyl Glycol, Xanthan Gum] marketed by BASF; Vitachelox [INCI: Vitis Vinifera Seed Extract, Camellia Sinensis Leaf Extract, Quercus Robur Wood Extract] marketed by Indena; L-VCG [INCI: Ascorbyl Glucoside]marketed by Freshine Bio-technology; Lumicease blue ingredient [INCI: Glycerin, Aqua, Hydrolyzed Pea Protein, Glucose, Sodium Chloride] marketed by Lipotec/Lubrizol; Lightwaves Defense [JS+M] [INCI: Jasminum Sambac Leaf Cell Extract] marketed by Naolys; Blue Oleoactif [INCI: Glycine Soja Oil, Polyglyceryl-3 Diisostearate, Oryza Sativa Germ Extract, Oryza Sativa Extract] marketed by Oleos-Hallstar; Majestem [INCI: Glycerin, Leontopodium Alpinum Callus Culture Extract, Xanthan Gum] or Senestem [INCI: Glycerin, Plantago Lanceolata Leaf Extract, Xanthan Gum] marketed by Sederma; Blueshield [INCI: Glycerin, Capsicum Annuum Fruit Extract, Xanthan Gum] marketed by Solabia; and combinations thereof.

In another embodiment, a DNA protecting agent, DNA repair agent, and/or stem cell protecting agent is selected from the group consisting of; GP4G SP [INCI: Aqua, Glycerin, Aretmia Extract], Heliostatine [INCI: Aqua, Glycerin, Pisum Sativum Extract], Orsirtine [INCI: Aqua, Glycerin, Oryza Sativa Extract], Chronogen [INCI: Aqua, Butylene Glycol, Tetrapeptide (INCI proposed)], Survixyl IS [INCI: Water, Butylene Glycol, Pentapeptide-31] and Chrondricare [INCI: Aqua, Butylene Glycol Pentapeptide-28] marketed by Vincience/ISP/Ashland; Lanacityn^{®} [INCI: Glycerin, Aqua, Alteromonas ferment extract, Chysanthellum indicum extract] or Melinoil [INCI: Isopropyl Palmitate, Lecithin, Aqua, Acetyl Hexapeptide-1] marketed by Atrium Innovations/Lucas Meyer Cosmetics; Repair Complex [INCI: Bifida Ferment Lysate] marketed by CLR; Phycojuvenine [INCI: Laminaria Digitata] marketed by Codif; Unirepair T-43 [INCI: Butylene Glycol, Acetyl Tyrosine, Proline, Hydrolyzed Vegetable Protein, Adenosine Triphosphate] marketed by Induchem; Dragosine [INCI: Carnosine] marketed by Symrise; DN-Age [INCI: Cassia Alata Leaf Extract] marketed by Laboratories Serobiologiques/Cognis/BASF; Helioguard [INCI: Porphyra Umbilicalis encapsulated into liposomes], PhytoCellTec Malus Domestica [INCI: PhytoCellTec Malus Domestica] or PhytoCellTec Argan [INCI: Argania Spinosa Sprout Cell Extraxt, Isomalt, Lecithin, Sodium Benzoate, Aqua] marketed by Mibelle Biochemistry; Pepha-Protect [INCI: Water Melon Extract] marketed by Pentapharm/DSM; Celligent [INCI: Helianthus Annuus Seed Oil, Ethyl Ferulate, Polyglyceryl-5 Trioleate, Rosmarinus Officinalis Leaf Extract, Aqua, Disodium Uridine Phosphate] or Defensil [INCI: Octyl Dodecanol, Echium Plantagineum Seed Oil, Cardiospermum Halicacabum Extract, Helianthus Annuus Seed Oil Unsaponifiables] marketed by Rahn; Venuceane [INCI: Thermus Thermophilus Ferment, Glycerin], UV-Soft [INCI: Yeast Extract], Renovage [INCI: Caprylic/Capric Triglyceride, Teprenone], Juvinity [INCI: Caprylic/Capric Triglyceride, Geranylgeranylpropanol (proposed)], Phytessence Holyherb [INCI: Butylene Glycol, Eriodictyon Californicum (Holyherb) Flower/Leaf/Stem Extract] or Resistem [INCI: Glycerin, Globularia Cordifolia Ferment] marketed by Sederma/Croda; Infraguard [INCI: Caesalpinia Spinosa Fruit Pod Extract, Propylene Glycol, Aqua, Helianthus Annuus Sprout Extract, Sodium Benzoate, Phenoxyethanol] marketed by Mibelle; Heliomoduline [INCI: Low molecular weight peptides from cottonseed] or Stem-C-Guard [Hydrolyzed Pea] marketed by Silab; and combinations thereof.

In another embodiment, the reactive carbonyl species scavenger, free radical scavengers and/or anti-glycation agent, detoxifying agent, antioxidant and/or anti-pollution agent is selected, for example and not restricted to, from the group formed by carnosine and its derivatives; GHK [INCI: Tripeptide-1] and its salts and/or derivatives or Quintescine IS [INCI: Dipeptide-4] marketed by Vincience/ISP/Ashland; Preregen [INCI: Glycine Soja (Soybean) Protein, Oxido Reductases], Edelweiss GC [INCI: Leontopodium Alpinum Extract], Lipogard [INCI: Squalane, Ubiquinone], Nectapure [INCI: Buddleja Davidii Extract, Thymus Vulgaris Extract], Alpaflor Nectapure [INCI: Buddleja Davidii Extract, Thymus Vulgaris Extract, Glycerin, Water] or Dismutin-BT [INCI: Highly purified SOD from a natural yeast strain of Saccharomyces cerevisiae] marketed by Pentapharm/DSM; Preventhelia^{®} [INCI: Diaminopropionoyl Tripeptide-33], Aldenine^{®} [INCI: Hydrolized Wheat Protein, Hydrolized Soy Protein, Tripeptide 1], Lipochroman^{™} [INCI: Dimethylmethoxy Chromanol], Thermostressine^{®} [INCI: Acetyl Tetrapeptide-22] Pollushield^{™} functional ingredient [INCI: Diisopropyl Adipate, Lecithin, Acrylic Acid/Acrylamidomethyl Propane Sulfonic Acid Copolymer, Dimethylmethoxy Chromanol, Xanthan Gum] or Bodyfensine^{®} [INCI: Acetyl Dipeptide-3 Aminohexanoate] marketed by Lipotec/Lubrizol; unactyl [INCI: Mannitol, Pisum Sativum Extract, Histidine HCl, Arginine, Cyclodextrin, Dextrin, Yeast Extract, Acetyl Trysoine, Pyridoxine HCl, Khaya Senegalensis Bark Extract, Nicotinamide, Adenine Dinucleotide, Disodium Succinate, Aspartic Acid], Imidinyl [INCI: Tamarindus Indica Seed Polysaccharide], Phystrogene [INCI: Butylene Glycol, Malva Sylvestris (Mallow) Extract, Xanthan Gum] or Purisoft [INCI: Moringa Pterogysperma Seed Extract] marketed by Laboratoires Sérobiologiques/Cognis/BASF; AquaCacteen [INCI: Glycerin, Opuntia Ficus Indica Stem Extract, Phenoxyethanol, Aqua], Trimoist (KMF) [INCI: Sodium Stearoyl Lactylate, Cetyl alcohol, Olus Vegetable oil, Tocopheryl acetate, Glycerin, Glycine soja sterol, Sodium lactate, Sodium barboxymethyl betaglucan, Carnosine, Lactic Acid], MelanoBronze [INCI: Vitex Agnus Castus Extract (Monk's pepper berries extract (phyto-endorphins)), Acetyl Tyrosine], CM-Glucan [INCI: Sodium Carobxymethyl Betaglucan, Phenoxyethanol, SunActin [INCI: Helianthus Annuus (Sunflower) Sprout Extract, Tocopherols, Glycerin, Lecithin, Phenoxyethanol, Aqua], GSP-T skin [INCI: Glycerin, Alcohol, Aqua, PEG-40 Hydrogenated Castor Oil, Vitis Vinifera (Grape) Seed Extract] or Detoxophane [INCI: Lepidium Sativum Sprout Extract, Lecithin, Phenoxyethanol, Glycerin, Water] marketed by Mibelle Biochemistry; Bacocalmine [INCI: PEG-8, Bacopa Monniera Extract, Water (Aqua), Hydroxyethylcellulose], Kombuchka [INCI: Saccharomyces/Xylinum Black Tea Ferment, Glycerin, Hydroxyethyl cellulose], Citystem [INCI: Glycerin, Marrubium Vulgare Extract] or Prodizia [INCI: Albizia Julibrissin Extract, Glycerin] marketed by Sederma/Croda; Extramel C [INCI: Hydroxypropyltrimonium Maltodextrin Crosspolymer, Cucumis Melo (Melon) Fruit Extract] marketed by Seppic; Defensine [INCI: Triticum Vulgare Germ Extract], Apolluskin^{®} [INCI: Taraxacum officinale (Dandelion) Extract], Detoxyl^{®} [INCI: Water, Butylene Glycol, Butyrospermum parkii (Shea Butter) Seedcake Extract] or Antiglyskin [INCI: Aqua, Helianthus Annuus Seed Extract] marketed by Silab; and combinations thereof.

The cosmetic composition of the invention can further include Oxygeskin^{®}, Tropaeolum majus [INCI: Tropaeolum majus Flower/Leaf/Stem Extract] from Silab.

The cosmetic composition of the invention can further include gamethophyte extracts. Non-limiting examples are Undaria pinnatifida extracts such as Emphemer^{™} from Seppic.

The cosmetic composition of the invention can further include Adipofillin^{™} by Lucas Meyer Cosmetics [INCI Name: Aqua (and) Propanediol (and) Ornithine (and) Phospholipids (and) Glycolipids].

The compositions of the invention may be for use in any of the applications or uses discussed under the heading "Applications".

### Applications

The invention is based on the finding that the botanical extract (the aqueous botanical extract and the purified aqueous botanical extract) and compositions described herein are useful in the cosmetic, non-therapeutic treatment of the skin, hair, nails and/or mucous membranes. In particular, they are useful in the cosmetic, non-therapeutic treatment and/or care of the skin. In the context of this invention, skin includes the skin of the whole body including the skin of the face (including skin around the eyes), neckline, neck, décolletage, arms, hands, legs, feet, thighs, hips, buttocks, stomach and torso, as well as the scalp (i.e. the area bordered by the human face at the front, and by the neck at the sides and back).

It has been found that, advantageously, the botanical extract of the invention is able to modulate the expression of Hypoxia-Inducible Factors (HIF) in human epidermal kerinatocytes. In particular, the botanical extract increases the expression of HIF-1α beyond the norm, causing an "overexpession" of HIF-1α. HIF-1α regulates the expression of vasodilatator signal molecules such as nitric oxide. Thus, in one embodiment the botanical extract of the invention increases HIF-1α expression in the skin. Also, it has been found that the botanical extract of the invention is able to downregulate the expression of HIF-2α in human epidermal kerinatocytes. Thus, in one embodiment, the botanical extract of the invention can also decrease or reduce HIF-2α expression in the skin, i.e. cause a reduction in the expression of HIF-2α beyond the norm or "downregulation" of HIF-2α. HIF-2α controls arginase enzyme expression which is known to compete with HIF-1α for use of the substrate, L-arginine. In one embodiment, the botanical extract of the invention increases HIF-1α expression and reduces HIF-2α expression in the skin. The botanical extract of the invention may increase HIF-1α expression and reduce HIF-2α expression, simultaneously, in the skin.

Surprisingly, it has also been found that the botanical extract of the invention is able to reduce or inhibit the activity of L-arginase enzyme in human epidermal kerinatocytes. It is known that L-Arginine is a common substrate for arginase and nitric oxide synthase enzymes. Without being bound by theory, it is belived that a reduction of L-Arginase enzyme activity may increase the bioavailability of L-arginine for the generation of vasodilation signalling molecules, such as nitric oxide. By increasing the vasodilation signalling molecules, the blood flow to the skin may increase, resulting in an increase in the transport of oxygen to the skin. Thus, in one embodiment the botanical extracts inhibit L-arginase activity in the skin. Therefore, the extract of the invention is able to increase skin or cutaneous oxygenation; and/or increase skin microcirculation and/or vascularization.

The botanical extract of the invention is useful in the cosmetic, non-therapeutic treatment or care of the skin, wherein the cosmetic, non-therapeutic treatment or care of the skin is the improvement or increase of skin oxygenation.

The botanical extract of the invention is useful in the cosmetic, non-therapeutic treatment or care of the skin, wherein the cosmetic, non-therapeutic treatment or care of the skin is the improvement and/or increase of skin microcirculation and/or vascularization.

The increase of vascularization of skin can increase skin rosiness. An increase in skin rosiness, also commonly referred to as natural flush, is an increase of the coloration of the skin or reddish complexion, which increases apparent health and attractiveness.

The botanical extract of the invention is useful in the cosmetic, non-therapeutic treatment or care of the skin, wherein the cosmetic, non-therapeutic treatment or care of the skin is the improvement and/or increase of skin rosiness (natural flush).

The botanical extract of the invention is useful in the cosmetic, non-therapeutic treatment or care of the skin, wherein the cosmetic, non-therapeutic treatment or care of the skin is the improvement or increase of skin glossiness and/or luminosity.

The botanical extract of the invention is useful in the cosmetic, non-therapeutic treatment or care of the skin wherein the cosmetic, non-therapeutic treatment or care of the skin is the reduction or prevention of the symptoms of skin aging. The symptoms of skin aging include the appearance of skin wrinkles, fine lines and skin microrelief, in particular.

The botanical extract of the invention is useful in the cosmetic, non-therapeutic treatment or care of the skin, wherein the cosmetic, non-therapeutic treatment or care of the skin is the reduction or decrease of skin roughness; and/or improvement or increase of skin smoothness.

Advantageously, the botanical extract of the invention not only exerts improved beneficial effects for the cosmetic, non-therapuetic treatment or care of the skin, but the treatment can also be devoid of side effects, such as those associated with the production of peroxynitrite. Peroxynitrite is a reactive molecule with high vasoconstriction and cytotoxic effects. By avoiding peroxynitrite production, the extract of the invention is able to reduce undesirable side effects such as vasoconstriction (which can reduce the vasodilation effect of nitric oxide) and cytotoxic effects in epidermal cells.

In one aspect, the invention provides for the use of the aqueous botanical extract or the purified aqueous botanical extract of the invention or a cosmetic composition comprising the aqueous botanical extract or the purified aqueous botanical extract of the invention for: improving or increasing skin oxygenation; improving and/or increasing skin microcirculation or vascularization; improving or increasing skin rosiness (natural flush); reducing and/or preventing the symptoms of skin aging; increasing skin smoothness; and/or improving or increasing skin glossiness and/or luminosity.

The invention provides the use of the aqueous botanical extract or the purified aqueous botanical extract of the invention or a cosmetic composition comprising the aqueous botanical extract or the purified aqueous botanical extract of the invention for improving or increasing skin oxygenation.

The invention provides the use of the aqueous botanical extract or the purified aqueous botanical extract of the invention or a cosmetic composition comprising the aqueous botanical extract or the purified aqueous botanical extract of the invention for improving and/or increasing skin microcirculation and/or vascularization.

The invention provides the use of the aqueous botanical extract or the purified aqueous botanical extract of the invention or a cosmetic composition comprising the aqueous botanical extract or the purified aqueous botanical extract of the invention for improving and/or increasing skin rosiness (natural flush).

The invention provides the use of the aqueous botanical extract or the purified aqueous botanical extract of the invention or a cosmetic composition comprising the aqueous botanical extract or the purified aqueous botanical extract of the invention for reducing or preventing the symptoms of skin aging.

The invention provides the use of the aqueous botanical extract or the purified aqueous botanical extract of the invention or a cosmetic composition comprising the aqueous botanical extract or the purified aqueous botanical extract of the invention for improving or increasing skin glossiness and/or luminosity.

The invention provides the use of the aqueous botanical extract or the purified aqueous botanical extract of the invention or a cosmetic composition comprising the aqueous botanical extract or the purified aqueous botanical extract of the invention for reducing or decreasing skin roughness; and/or improving or increasing skin smoothness.

In another aspect, the invention provides a method of treatment and/or care of the skin of a subject comprising administering the aqueous botanical extract of the invention, the purified aqueous botanical extract of the invention or a cosmetic composition comprising the aqueous botanical extract or the purified aqueous botanical extract of the invention to the subject. In particular, the invention provides a cosmetic, non-therapeutic method of treatment and/or care of the skin of a subject comprising administering a cosmetically effective amount of the aqueous botanical extract of the invention, the purified aqueous botanical extract of the invention or a cosmetic composition comprising the aqueous botanical extract or the purified aqueous botanical extract of the invention to the subject. The method can be for the treatment and/or care of the skin as described above in relation to the applications (uses) of the compounds and compositions of the invention. The administration can be topical or, for example, transdermal. In this aspect of the invention, the botanical extracts of the invention may be present in a cosmetic composition such as the cosmetic compositions as described herein. In one embodiment, the method involves administering the compound or administering the composition using microneedles.

The above methods of treatment include the cosmetic, non-therapeutic treatment and/or care of the skin, including: the improvement or increase of skin oxygenation; the improvement and/or increase of skin microcirculation or vascularization; the improvement or increase of skin rosiness (natural flush); the reduction and/or prevention of the symptoms of skin aging; the increase of skin smoothness; and/or the improvement or increase of skin glossiness and/or luminosity.

In another aspect, the invention provides the aqueous botanical extract or the purified aqueous botanical extract of the invention, for use as a medicament. In particular, the invention provides the aqueous botanical extract or the purified aqueous botanical extract of the invention, for use in the treatment or prevention of a disease or disorder. In another aspect, the invention provides for the use of the aqueous botanical extract or the purified aqueous botanical extract of the invention in the manufacture of a medicament for the treatment or prevention of a disease or disorder. In another aspect, the invention provides a method of treating or preventing a disease or disorder in a subject comprising administering a therapeutically effective amount of the aqueous botanical extract or the purified aqueous botanical extract of the invention or a pharmaceutical composition comprising same, to the subject.

For the above described methods of the invention, topical or transdermal application can be carried out by iontophoresis, sonophoresis, electroporation, mechanical pressure, osmotic pressure gradient, occlusive cure, microinjections, by needle-free injections by means of pressure, by microelectric patches, face masks or any combination thereof.

For the above described methods of the invention, the frequency of application or administration can vary greatly, depending on the needs of each subject, with a recommendation of an application from once a month to ten times a day, preferably from once a week to four times a day, more preferably from three times a week to twice a day, even more preferably once a day.

The invention is further described in the following non-limiting examples.

### EXAMPLES

### EXAMPLE 1

### Subcritical Water Extraction from Liqustrum lucidum fruit at 160°C

*Ligustrum lucidum* fruit were obtained from Sichuan, China. The fruit had a brownish dried form. Before extraction, the raw material was crushed to an average particle size corresponding to 3 mm mesh. The ground fruit were stored at room temperature until further use. Subcritical water extraction was performed using an accelerated solvent extractor (ASE350, Dionex, USA) and using the following procedure: 7.6g of the *Ligustrum lucidum* dried fruit and 10 g of diatomaceous earth (ASE prep DE, Dionex) were loaded into a 100mL stainless steel cell. The diatomaceous earth acts as a dispersant and a drying agent. The cell was placed in the extractor, and water was added to fill the cell and then heated to 160°C under a pressure set at 1.38 MPa. Extraction was perfomed for 5 minutes and the extract collected in a collection bottle. The cell was filled again with water and the extraction conducted in the same conditions for 2 additional cycles of 5 minutes. The total extraction time was 15 minutes (3 cycles of 5 minutes). The cell was then purged for 10 minutes to obtain the residual water remaining inside. The extracts obtained in each of the cycles as well as the residual water were pooled together in the same collection bottle. The total amount of water used was about 170 g. The aqueous extract was mixed with 100% glycerin to make a 55% glycerin solution (on a weight basis). The ratio of raw material (dried fruit) to the 55% glycerin solution was 1:49 by weight.

### EXAMPLE 2

### Subcritical water extraction from Liqustrum lucidum fruit at 160°C

An aqueous extract was obtained as described in Example 1, except that 5 g of raw material (i.e. dried fruit) and the total amout of water used was about 110 g. Glycerin 100% was added to the aqueous extract to make an 80 % glycerin solution (on a weight basis) and obtain a final ratio of raw material (i.e. dried fruit) to the 80% glycerin solution of 1:100.

### EXAMPLE 3

### Subcritical water extraction from Liqustrum lucidum fruit at 125°C

An aqueous extract from dried *Ligustrum lucidum* fruit was obtained as described in Example 1, except that the cell was heated to 125°C. The aqueous extract obtained was mixed with 100% glycerin so as to make a 55% glycerin solution and to obtain a ratio of raw material (i.e. dried fruit) to the 55% glycerin solution of 1:49 by weight.

### EXAMPLE 4

### Extraction from Ligustrum lucidum fruit with 50% glycerin (w/w)

Dried and ground *Ligustrum lucidum* fruit were mixed with a 50% glycerin solution (by weight) in a ratio of 1:53 (dried fruit: 50% glycerin solution, by weight). The extraction was performed at 60°C for 30 minutes while stirring. Subsequently, fruit and 50 % glycerin solution mixture was allowed to cool at room temperature, and was filtered with 10µm filter.

### EXAMPLE 5

### Extraction from Ligustrum lucidum fruit with 80% glycerin (w/w)

Extraction was performed as described in Example 4, except an 80% glycerin solution (by weight) was used and the ratio of dried fruit to the 50 % glycerin solution which was 1:45.

### EXAMPLE 6

### Cold extraction from Ligustrum lucidum fruit with water

Dried and ground *Ligustrum lucidum* fruit were mixed with distilled water in a ratio of 1:48 (dried fruit: water, by weight). The mixture was put into a refrigerator (4°C) for 72 hours. Subsequently, the extract was filtered through 10µm filter.

### EXAMPLE 7

### Ethanolic extraction from Liqustrum lucidum fruit

Dried and ground *Ligustrum lucidum* fruit were mixed with 70% ethanol (w/w) in a ratio of 1:141 (dried fruit: 70% ethanol, by weight). Extraction was performed at room temperature for 7 days with stirring. The extract was filtered through 10µm filter.

### EXAMPLE 8

### Ethanolic extraction from Liqustrum lucidum fruit

Extraction was conducted as described in Example 7, except that a ratio of 1:50 (dried fruit: 70% ethanol, by weight) was used.

The conditions for each extraction method are summarized below.

**Table 2: Extraction conditions for Examples 1 to 8**

| Example | Extraction (description) | Extraction Temperature (°C) | Extraction time | Raw material (dried fruit): liquid (w/w) |
|---|---|---|---|---|
| 1 | **SWE 160°C GL55** (Subcritical water | 160 | 15 min. | 1:49 (55 % glycerin |
| | extraction at 160°C) | | | solution) |
| 2 | **SWE 160°C GL80**(Subcritical water extraction at 160°C) | 160 | 15 min. | 1:100 (80 % glycerin solution) |
| 3 | **SWE 125°C GL55** (Subcritical water extraction at 125°C) | 125 | 15 min. | 1:49 (55 % glycerin solution) |
| 4 | **CE_GL50** (Conventional extraction with glycerin 50% solution (w/w)) | 60 | 48hrs | 1:53 (50% glycerin solution) |
| 5 | **CE_GL80** Conventional extraction with glycerin 80% solution (w/w) | 60 | 48hrs | 1:45 (80% glycerin solution) |
| 6 | **Cold extraction** (Water extraction at 4°C) | 4 | 72hrs | 1:48 (water) |
| 7 | **EtOH70** (70% (w/w) ethanol) | 25 | 7d | 1:141 (70 % ethanol) |
| 8 | **EtOH70** (70% (w/w) ethanol) | 25 | 7d | 1:50 (70 % ethanol) |

### EXAMPLE 9

### Phenolic content analysis

Total polyphenol content (TPC) and LC/MS-MS (liquid chromatography with tandem mass spectrometry) analyses were conducted for each extract obtained in Examples 1 and 4-8. The TPC was determined by Folin-Ciocalteu method. Briefly, 2mL of extract was mixed with 5mL of distilled water and then 1mL of Folin-Ciocalteu reagent was added to the mixture and the mixture was mixed well for 3 min. After 3 min, 5mL of 10% sodium carbonate solution was added to stop the reaction. After 30 minutes at room temperature, absorbance was measured at 760 and 850 nm using a spectrophotometer. Gallic acid was used as a standard to obtain a calibration curve.

LC-MS-MS was used for identifying more specific phytochemicals. Ten microlitres (10 µL) of extract were mixed with 100µL of 10mM HCL and 890µL of ultrapure water in a 2mL LC-MS vial, the samples were diluted 100 times. Injection volume was 2 µL. The mobile phase used for this analysis comprised 0.1% formic acid in acetonitrile (solvent A) and 0.1% of formic acid in ultrapure water (Solvent B). The flow rate was 0.2 mL/min at 40°C column temperature.

**Table 3: Quantified bio-actives in Ligustrum lucidum obtained in examples 1, 4 to 8. The results are expressed in ppm. ND = none detected.**

| | Ex. 1 | Ex. 4 | Ex. 5 | Ex. 6 | Ex. 7 | Ex. 8 |
|---|---|---|---|---|---|---|
| | SWE160 GL55 | CE_GL5 0 | CE_GL8 0 | Cold Extractio n | EtOH70 | EtOH70 |
| vanillin | 0.426 | ND | ND | ND | ND | ND |
| Luteolin-7-O-glucoside | 0.757 | 0.435 | 0.624 | 0.130 | 0.326 | 0.379 |
| 3,4/3,5-dihydroxybenzoic acid | 0.843 | 1.156 | 1.882 | 0.948 | ND | ND |
| 4-hydroxybenzaldehyde | 0.093 | ND | ND | ND | ND | ND |
| 4-Hydroxybenzoic acid | 0.221 | ND | ND | ND | ND | ND |
| vanillic acid | 0.204 | 0.243 | 0.301 | 0.342 | 0.167 | 0.237 |
| caffeic acid | 0.371 | 0.147 | 0.246 | 0.284 | ND | ND |
| ferulic acid | 0.076 | 0.029 | 0.041 | ND | 0.018 | 0.034 |
| Abscisic acid | 0.005 | ND | ND | ND | 0.004 | 0.005 |
| Quercetin | 0.063 | ND | ND | ND | ND | ND |
| Eriodictyol | 0.009 | 0.008 | 0.006 | ND | 0.028 | 0.019 |
| Sinapyl-alcohol | 0.978 | ND | ND | ND | ND | ND |
| Salicylic acid | 0.156 | ND | ND | ND | ND | ND |
| Coniferaldehyde | 0.169 | 0.053 | 0.069 | 0.071 | 0.024 | 0.048 |
| Apigenin-7-O-glucoside | 0.158 | 0.113 | 0.171 | 0.039 | 0.108 | 0.135 |
| Cinnamic acid | 0.317 | 0.255 | 0.265 | 0.652 | ND | 0.121 |
| Coniferyl-alcohol | 8.666 | ND | ND | ND | ND | 1.009 |
| Coumaric acid | 0.564 | 0.104 | 0.167 | ND | 0.028 | 0.108 |
| Myricetin | 1.223 | ND | ND | ND | ND | ND |
| Kaempferol | 0.234 | 0.099 | 0.054 | 0.068 | ND | ND |
| 3,5-dihydroxybenzaldehyde | 0.202 | ND | ND | ND | 0.339 | 0.076 |
| Kaempferol-3-O-glucoside | ND | ND | ND | ND | 0.014 | 0.012 |
| Rutin | ND | ND | ND | ND | 0.237 | 0.230 |
| Luteolin | ND | ND | ND | ND | 0.304 | 0.154 |
| 3,4 & 3,5-dihydroxybenzoic acid (co-ellute) | ND | ND | ND | ND | 0.411 | 0.558 |
| 2,5-dihydroxybenzoic acid | ND | ND | ND | ND | 0.202 | 0.27 |
| alpha-Amyrin | ND | ND | ND | ND | 0.046 | ND |
| Naringenin | 0.003 | ND | ND | ND | ND | ND |
| Apigenin | 2.266 | ND | ND | ND | 0.671 | 0.844 |
| Oleuropein | 24.519 | 22.493 | 16.205 | 0.908 | 13.352 | 20.867 |
| Salidroside | 19.071 | 18.946 | 6.954 | 1.361 | 2.464 | 19.967 |
| Total phenolics | 61.594 | 44.081 | 26.985 | 4.803 | 18.744 | 45.073 |

### EXAMPLE 10

### Determination of maximum concentrations of extract to be tested in Human epidermal keratinocyte cells (HEK).

Human epidermal keratinocytes (HEK) in culture continuously divide and multiply over time. A compound interfering this process reduces growth rate and can provoke cell morphology alteration. As a result, a decrease of cell number is observed and the viability of HEK culture is decreased.

Neutral red (NR) uptake assay procedure is a cell viability assay based on the ability of viable cells to incorporate and bind NR, a supravital dye. NR is a weak cationic dye that penetrates cell membranes by non-ionic diffusion and accumulates intracellularly in lysosomes. Alterations of the cell surface or the sensitive lysosomal membrane lead to lysosomal fragility and other changes that gradually become irreversible. Such changes result in a decreased uptake and binding of NR. It is thus possible to distinguish between viable, damaged, or dead cells after microscope observation. Viability in this test is expressed as a concentration-dependent reduction of the uptake of the vital dye NR when measured 48-hours after treatment with the test compounds.

HEK (Tebu-BIO) were seeded in 96-well plates at a density of 2x10⁴ cells/well in keratinocyte growth medium supplemented with Mix C39016-CaCl₂ solution (Promocell). After 24 hours of incubation, the medium was replaced with fresh culture medium with extracts of Example 1, 3, 4, 5 or 6. Treatment was continued for 48 hours and non-treated cells were used as basal control. After 48 hours treatment, cells viability was determined by NR uptake by incubating for 2 hours with a 50 µg/ml NR (Sigma) solution in culture medium. After 1 hour, cells morphology was observed. At the end of 2 hours incubation, NR staining was desorbed with a Desorb solution (ethanol:water:acetic acid 50:49:1 v/v). Finally, plates were shaken for 10 minutes and the optical density of NR extract was measured at 540 nm using a microplate reader (Clariostar, BMG). Viability percentage of each condition was normalized to basal condition.

The results below are expressed viability percentage normalized by basal condition and cell morphology condition.

**Table 4: Mean values of viability percentage with respect to basal condition, and morphology observation of HEK cells compared to basal condition cells (N = normal morpohology, A = altered morphology)**

| | | Dose % (v/v) | | | | | |
|---|---|---|---|---|---|---|---|
| | | 0.1 | | 0.5 | | 1 | |
| Extract of | Type of extraction | Viability (%) | Morphology | Viability (%) | Morphology | Viability (%) | Morphology |
| Example 3 | Subcritical water 125°C | 102.1 | N | 105.0 | N | 111.7 | N |
| Example 1 | Subcritical water 160°C | 102.9 | N | 100.8 | N | 107.7 | N |
| Example 5 | GL80 | 95.81 | A | 93.15 | A | 88.95 | A |
| Example 4 | GL50 | 104.6 | N | 92.04 | A | 85.96 | A |
| Example 6 | Cold Extraction | 94.87 | N | 95.38 | N | 97.62 | A |

The extracts of Example 1 and 3 (subcritical water extraction) did not alter the viability or the morphology of the cells, denoting a low toxicity for these extracts. In contrast, the glycerin extracts of Examples 4 and 5 reduced the viability of the cells more than 10% at the highest concentration (1% of extract v/v) and altered cell morphology in concentrations of 0.5% (v/) or higher. In general terms, reductions of viability higher than 10% are considered not acceptable for further efficacy tests. These results demonstrate a higher deleterious effect of the glycerin extracts which limits the maximum tolerable dosage allowable for said extracts.

### EXAMPLE 11

### In vitro relative quantification of HIF isoforms gene expression by quantitative-Polymerase chain reaction.

Human epidermal keratinocytes (HEK)(Tebu-BIO) were seeded in 12-well plates at a density of 3x10⁵ cells/well in keratinocyte growth medium supplemented with Mix C39016-CaCl₂ solution (Promocell). After 24 hours incubation, the medium was replaced with fresh culture medium with extracts of Example 1, 3, 4, 5 or 6. The concentration of extracts used was determined according to Example 10. Treatment was continued for 48 hours and cells treated with vehicle (i.e. the same solvent of the extract) were used as basal control. After 48 hours of treatment, an RNA extraction was performed using RNeasy mini kit (Qiagen) following the manufacturer's protocol. The RNA of each test item and vehicle was then quantified and its purity was analyzed with a Nanodrop (Thermo). After that, 2 µg of each RNA sample were retrotranscribed with iScript Advanced (*BioRad*) in a final volume of 20 µL. The RNA sample and the iScript Advanced were incubated in a thermal cycler (*Eppendorf*) at 42°C for 30 minutes and the reaction was stopped at 85°C for 5 minutes.

Complementary DNA was amplified by quantitative Polymerase chain reaction (qPCR) in a real-time PCR thermocycler *(BioRad)* using SYBR Green Supermix *(BioRad)* in a 96-well panel for use with SYBR Green *(BioRad).* Cycling conditions in BioRad CFX96 instrument were 95°C for 3 minutes, followed by 40 cycles of denaturing at 95°C for 5 seconds, annealing and elongation at 60°C for 30 seconds. Glyceraldehyde 3-phosphate dehydrogenase (GAPDH), and Hypoxanthine phosphoribosyltransferase 1 (HRPT1) were used as endogenous controls. Fold change relative to the expression of the sample HIF1 and HIF2 (*EPAS1*) genes and reference genes was calculated using normalized expression (ΔΔ(Ct)) method using CFX Manager Software *(BioRad).*

The results are expressed as a percentage of gene expression increase/reduction normalized by the corresponding vehicle for each extraction.

**Table 6: Mean and standard error of the mean (SEM) values of HIF1 and HIF2 expression with respect to the vehicle of each extraction. Statistical significance calculated using an unpaired Student's t-test. **: p<0.01; n.s. = not significant, p>0.05**

| Extract of | Type of extraction | Dose % (v/v) | HIF1 | | | HIF2 | | |
|---|---|---|---|---|---|---|---|---|
| | | | Mean | SEM | stat | Mean | SEM | stat |
| Example 3 | Subcritical water 125°C | 1.0 | 86.6 | 19.7 | n.s | 75.9 | 24.6 | n.s |
| Example 1 | Subcritical water 160°C | 1.0 | 114.0 | 5.2 | ** | 83.6 | 3.7 | ** |
| Example 5 | GL80 | 0.5 | 101.1 | 5.2 | n.s | 59.3 | 24.1 | ** |
| Example 4 | GL50 | 0.5 | 86.5 | 13.0 | n.s | 99.4 | 5.8 | n.s |
| Example 6 | Cold extraction | 1.0 | 107.5 | 6.8 | n.s | 110.2 | 9.1 | n.s |

**Table 7: Percentage of variation of HIF1 and HIF2 for each extraction vs vehicle. Statistical significance calculated using an unpaired Student's t test. **: p<0.01; n.s. = not significant, p>0.05**

| Extract of | Type of extraction | Dose % (v/v) | *HIF1* | | *HIF2* | |
|---|---|---|---|---|---|---|
| | | | % increase | stat | % decrease | stat |
| Example 3 | Subcritical water 125°C | 1.0 | -13.4 | n.s | -24.1 | n.s |
| Example 1 | Subcritical water 160°C | 1.0 | 14.0 | ** | -16.4 | ** |
| Example 5 | GL80 | 0.5 | 1.1 | n.s | -40.7 | ** |
| Example 4 | GL50 | 0.5 | -13.5 | n.s | -0.6 | n.s |
| Example 6 | Cold extraction | 1.0 | 7.5 | n.s | 10.2 | n.s |

The results demonstrate that *Ligustrum lucidum* extraction using subcritical water at 160°C provides the only extract able to increase HIF1 expression levels and simultaneously decrease HIF2 expression levels. This effect was not seen in any of the other extracts tested. Also, the extracts obtained using subcritical water extraction at 160°C show better results in comparison to the other plant extraction methodologies tested.

### EXAMPLE 12

### Evaluation of arginase activity reduction in human epidermal keratinocytes.

L- Arginine is a common substrate for arginase and nitric oxide synthase enzymes. Depending on their equilibrium, different effects can be observed: nitric oxide synthase will lead to the production of vasodilating signals such as nitric oxide, whereas arginase will metabolize L-arginine into urea and ornithine. For this reason, compounds able to decrease arginase activity will allow a higher bioavailability of L-arginine for the generation of vasodilation signaling and, therefore, will enhance skin oxygenation status.

Human epidermal keratinocytes (HEK) of donor of 54 years in age (Tebu Bio), donor of 36 years in age (Promocell) and donor of 26 years in age (Promocell) were seeded in 6-well plates at a density of 3x10⁵ cells/well in keratinocyte growth medium supplemented with Mix C39016-CaCl₂ solution (Promocell). After 24 hours incubation, medium was replaced with fresh culture medium with scalar dilutions of the subcritical water extract of Example 2 or the ethanolic extract of Example 7. The 54-year-old donor's cells treated with S-(2-Boronoethyl)-L-cysteine hydrochloride (BEC, Sigma) 1 mM were used as positive control of arginase inhibition, and the 54-year-old donor's, 36-year-old donor's and 26-year-old donor's cells treated with medium alone were used as basal control of arginase activity at different ages. Treatment was continued for 24 hours. After 24 hours, cells were lysed and were analyzed by a colorimetric Arginase Activity kit (Abcam) according to manufacturer's instructions. In brief, arginase reacts with arginine and undergoes a series of reactions to form an intermediate that reacts stoichiometrically with the probe leading to the appearance of a colored product. Absorbance is measured by using a microplate reader (Clariostar, BMG) set to 570 nm in a kinetic mode for 30 minutes. A time-dependent curve was obtained with the lecture. Arginase activity was calculated in the exponential phase of the kinetic for each sample with the following equation: ΔOD = (OD2 - ODBG2) - (OD1 - ODBG1); where: OD1 and OD2 was the sample reading at time T1 and T2 respectively, ODBG1 and ODBG2 was the sample background reading at time T1 and T2 respectively.

Total protein amount of each sample was determined using a Pierce BCA protein assay kit (Thermo fisher) according to the manufacturer's protocol. Briefly, after adding Working Reagent to the samples and the standards, the samples were incubated. Afterwards, color change was measured with an absorbance microplate reader (Clariostar, BMG) at 562 nm. The total protein amount was used to normalize the level of arginase activity obtained by the Arginase activity test in the samples.

The results are expressed as a percentage of arginase activity normalized by 54-year-old basal condition.

**Table 8: Mean and SEM values of arginase activity respect basal condition of 54-year-old cells. Statistical significance calculated using an unpaired Student's t test. *: p<0.05 **: p<0.01 ***: p<0.001 ****: p<0.0001; ns: Not statistically significant**

| Condition | MEAN | SEM | Stat. |
|---|---|---|---|
| Basal condition 54-year-old | 100.00 | 0.57 | |
| Basal condition 36-year-old | 78.80 | 2.91 | **** |
| Basal condition 26-year-old | 72.97 | 1.95 | **** |
| BEC 1mM 54-year-old | 70.10 | 1.91 | **** |
| *L. lucidum* SWE of Example 2 at 0.05 % (v/v) 54-year-old | 88.37 | 2.86 | **** |
| *L. lucidum* SWE of Example 2 at 0.005 % (v/v) 54-year-old | 95.98 | 1.26 | ** |
| *L. lucidum* EtOH of Example 7 at 0.062 % (v/v) 54-year-old | 112.30 | 2.62 | * |
| *L. lucidum* EtOH of Example 7 at 0.05 % (v/v) 54-year-old | 99.95 | 3.75 | ns |
| *L. lucidum* EtOH of Example 7 at 0.005 % (v/v) 54-year-old | 103.70 | 0.55 | ns |

The results confirm that arginase activity increases with aging in epidermal cells as the arginase activity was higher for the 54-year-old's cells than for the 36-year-old's cells. Treatment with the subcritical water extract from *Ligustrum lucidum* of the invention decreases arginase activity in the 54-year-old's cells, which should make more arginine available to be used as a substrate by nitric oxide synthase thus, resulting in higher production of nitric oxide and better skin oxygenation.

The results for the SWE extract can be also expressed as a percentage of arginase activity when subtracting the 26-year-old basal condition and normalizing by 54-year-old basal condition

**Table 9: Mean and SEM values of arginase activity subtracting 26-year-old basal condition and normalizing respect basal condition of 54-year-old's cells. Statistical significance calculated using an unpaired Student's t test. **: p<0.01 ***: p<0.001 ****: p<0.0001**

| Condition | MEAN | SEM | Stat. |
|---|---|---|---|
| Basal condition 54-year-old | 100.00 | 2.11 | |
| Basal condition 36-year-old | 21.48 | 10.72 | **** |
| Basal condition 26-year-old | 0.00 | | |
| BEC 1mM 54-year-old | -10.58 | 7.07 | **** |
| *L. Lucidum* of Example 2 at 0.05 % (v/v) 54-year-old | 56.71 | 10.54 | **** |
| *L. Lucidum* of Example 2 at 0.005 % (v/v) 54-year-old | 84.75 | 4.66 | ** |

### EXAMPLE 13

In vitro quantification of peroxynitrite production in Human epidermal keratinocytes.

During oxidative stress-related states, ROS (reactive oxygen species) accumulate inside the cells and the detoxification repair mechanisms are not enough to maintain cell viability. Under these circumstances, ROS are involved in the peroxidation of lipids, amino acid oxidation and DNA damage, three processes which contribute to aging.

Vasodilation signaling molecule production can lead to a concomitant production of the by-product peroxynitrite, due to the reaction of nitric oxide with ROS. Peroxynitrite is a potent vasoconstrictor and toxic compound inside cells. Peroxynitrite acts as a deleterious molecule which can mask the beneficial effect of an enhanced vasodilator signaling in the skin. It is desirable to have products that are able to improve vasodilation signaling while avoiding peroxynitrite production.

Human epidermal keratinocytes (HEK) were seeded in 96-well plates at a density of 3x10⁴ cells/well in keratinocyte growth medium supplemented with Mix C39016-CaCl₂ solution (Promocell). After 24 hours incubation, medium was replaced with fresh culture medium with scalar dilutions of the subcritical water extract at 160°C of Example 2. Treatment was continued for 24 hours and cells treated with medium alone were used as basal control. After 24 hours incubation, peroxynitrite production was measured by a fluorescence approach with Peroxynitrite assay kit (Abcam) following the manufacturer's instructions. In brief, cells were treated with 10 µl/well sensor green working solution during 1 hour in the medium. Cells treated with SIN-1 (Sigma) 11 mM or 5.76 mM during 1 hour in combination with sensor green solution were used as a positive control of peroxynitrite production. SIN-1 (Sigma) is a nitric oxide donor. After 1-hour incubation, fluorescence was measured with an absorbance microplate reader (Clariostar, BMG) at excitation 490 nm and emission 530 nm. Finally, cell nuclei were stained by adding 50 µl/well of Hoescht 1/1000 (Thermo Fischer) to each well for 10 minutes. Cell nuclei number was determined with a confocal microscopy Operetta (PerkinElmer). Peroxynitrite fluorescence intensity was normalized to cell number for each condition.

The results are expressed as a percentage of peroxynitrite per cell normalized by basal condition.

**Table 10: Mean and SEM values of peroxynitrite production respect basal condition. Statistical significance calculated using an unpaired Student's t test. ****: p<0.0001**

| Condition | MEAN | SEM | Stat. |
|---|---|---|---|
| Basal condition | 100.00 | 0.97 | |
| SIN-1 5,76 mM | 2379.00 | 432 | **** |
| SIN-1 11 mM | 1654.00 | 224.5 | **** |
| *Ligustrum lucidum* 0.1 % (v/v) | 95.74 | 2.83 | n.s |
| *Ligustrum lucidum* 0.05 % (v/v) | 97.07 | 2.57 | n.s |
| *Ligustrum lucidum* 0.005 % (v/v) | 100.30 | 2.75 | n.s |

The results demonstrate that treatment with the extract from *Ligustrum lucidum* of Example 2 does not increase the production of peroxynitrite, and thus will avoid the appearance of the vasoconstriction signal in old epidermal cells.

### EXAMPLE 14

### In vitro evaluation of quantification of peroxynitrite production in Human epidermal keratinocytes treated with SIN-1.

Cellular ROS levels increase during aging and in response to different external sources including exposition of chemicals, ionizing radiation and bacterial or viral infections. Increased ROS production combined with augmented nitric oxide during aging, trigger the production of peroxynitrite, a by-product compound with a vasoconstriction and toxic effect. In this sense, products able to decrease peroxynitrite levels, which increase during aging, would lead to a better vasodilation signaling effect in skin cells.

Human epidermal keratinocytes (HEK) were seeded in 96 well plates at a density of 3x10⁴ cells/well in keratinocyte growth medium supplemented with Mix C39016-CaCl₂ solution (Promocell). After 24 hours incubation, medium was replaced with fresh culture medium with scalar dilutions of *Ligustrum Lucidum* extract obtained with subcritical water 160°C extraction according to Example 2. Treatment was continued for 24 hours and cells treated with medium alone were used as basal control. After 24 hours of incubation, peroxynitrite levels were measured by a fluorescence approach with Peroxynitrite assay kit (Abcam) following the manufacturer's instructions. In brief, cells were treated with 10 µl/well sensor green working solution and SIN-1 (Sigma) 20 mM for 1 hour. SIN-1 is a powerful oxidant compound which form peroxynitrite inside the cells, mimicking the situation which take place during aging [Hogg, N. "Production of hydroxyl radicals from the simultaneous generation of superoxide and nitric oxide" (1992). Biochemical Journal. 291, 419-424]. After 1-hour incubation, cells were washed with PBS and cell nuclei were stained with hoescht 1/1000 (Thermo Fischer) for 10 minutes. Finally, peroxynitrite fluorescence intensity and cell nuclei were determined with a confocal microscopy Operetta (PerkinElmer). Peroxynitrite fluorescence intensity was normalized to cell number for each condition.

The results are expressed as a percentage of peroxynitrite per cell normalized by basal condition treated with SIN-1 20 mM.

**Table 11: Mean and SEM values of peroxynitrite levels respect basal condition treated with SIN-1 20 mM. Statistical significance calculated using an unpaired Student's t test. *: p<0,05 **: p<0,01 ***: p<0,001 ****: p<0,0001**

| Condition | MEAN | SEM | Stat. |
|---|---|---|---|
| Basal condition | 46.82 | 5.09 | **** |
| Basal condition + SIN-1 20mM | 100.00 | 3.82 | |
| *Ligustrum Lucidum* 0.005 % (v/v) + SIN 20mM | 98.17 | 2.72 | n.s |
| *Ligustrum Lucidum* 0.05 % (v/v) + SIN 20mM | 91.61 | 2.36 | n.s |
| *Ligustrum Lucidum* 0.1 % (v/v) + SIN 20mM | 84.59 | 5.71 | * |
| *Ligustrum Lucidum* 0.5 % (v/v) + SIN 20mM | 76.08 | 4.83 | ** |

The results demonstrate that the extract from *Ligustrum lucidum* of Example 2 can reduce peroxynitrite levels in epidermal cells leading to a reduction of the vasoconstriction and toxic signal associated to aging.

### EXAMPLE 15

### Sprayable serum

In a suitable vessel, the ingredients of phase A are weighted: water [INCI: WATER (AQUA)].

Phase B1: Pemulen^{™} EZ-4U polymeric emulsifier [INCI: ACRYLATES/ C10/30 ALKYL ACRYLATE CROSSPOLYMER] is added in the previous mixture under stirring. Once dispersed, phase B2: Zemea^{®} [INCI: PROPANEDIOL], Glycerin [INCI: GLYCERIN], is added to the previous mixture and mixed until homogeneous.

The previous mixture is heated at 35°C and phase B3: dermosoft^{®} GMCY MB [INCI: GLYCERYL CAPRYLATE] is added under mixing.

Finally phase B4: RonaFlair^{®} LDP White [INCI: SODIUM POTASSIUM ALUMINUM, SILICATE, SILICA, TITANIUM DIOXIDE] is added and mixed with moderate stirring until homogeneous.

In a separate vessel, phase C ingredients: Schercemol^{™} 1818 Ester [INCI: ISOSTEARYL ISOSTEARATE], Cosphaderm^{®} TOM RSPO MB [INCI: PENTYLENE GLYCOL, GLYCERYL CAPRYLATE, GLYCERYL UNDECYLENATE] are mixed. The emulsion is made by adding slowly phase C to the previous mixture under fast stirring with a turbine.

pH is adjusted to 5.3 - 5.8 with phase D ingredient: Sodium Hydroxide 20% w/w [INCI: WATER (AQUA); SODIUM HYDROXIDE])

Phase E: Ligustrum lucidum subcritical water extract according to Example 2 [INCI: GLYCERIN, LIGUSTRUM LUCIDUM SEED EXTRACT] is added to the previous mixture.

Phase F: water [INCI: WATER (AQUA)], Dissolvine^{®} NA2-P [DISODIUM EDTA], is premixed and added to the previous misture under moderate stirring.

**Table 12**

| Phase | INGREDIENT (INCI name) | % weight |
|---|---|---|
| A | WATER (AQUA) | 81.55 |
| B1 | ACRYLATES/C10-30 ALKYL ACRYLATE CROSSPOLYMER | 0.2 |
| B2 | PROPANEDIOL | 5.0 |
| B2 | GLYCERIN | 5.0 |
| B3 | GLYCERYL CAPRYLATE | 1.0 |
| B4 | [SODIUM POTASSIUM ALUMINUM SILICATE, SILICA, TITANIUM DIOXIDE] | 0.5 |
| C | ISOSTEARYL ISOSTEARATE | 2.0 |
| C | [PENTYLENE GLYCOL, GLYCERYL CAPRYLATE, GLYCERYL UNDECYLENATE] | 1.6 |
| D | [WATER (AQUA), SODIUM HYDROXIDE] | 0.1 |
| E | [GLYCERIN, LIGUSTRUM LUCIDUM SEED EXTRACT] | 2.0 |
| F | WATER (AQUA) | 1.0 |
| F | DISODIUM EDTA | 0.05 |

### EXAMPLE 16

### Make-up fixer solution comprising Ligustrum lucidum subcritical water extract

In a suitable vessel, the ingredients of phase A are weighted: water [INCI: WATER (AQUA)], Carbopol^{®} Aqua SF-1 OS polymer [INCI: WATER (AQUA), ACRYLATES COPOLYMER], Novethix^{™} L-10 polymer [INCI: WATER (AQUA), ACRYLATES/BEHENETH-25 METHACRYLATE COPOLYMER], one by one under stirring.

Then phase A is neutralized using phase B: Sodium Hydroxide 20% w/w [INCI: WATER (AQUA); SODIUM HYDROXIDE], with slow stirring, taking care to not incorporate air bubbles.

In a separate vessel, phase C: RonaFlair^{®} Balanced Gold [TITANIUM DIOXIDE (CI 77891), MICA, TIN OXIDE], RonaFlair^{®} Balanced Red [TITANIUM DIOXIDE (CI 77891), MICA, TIN OXIDE], Colorona^{®} Oriental Beige [INCI: MICA, TITANIUM DIOXIDE (CI 77891), IRON OXIDES (Cl 77491)], Ronastar^{®} Frozen Jewel [INCI: CALCIUM ALUMINUM BOROSILICATE, SILICA, TITANIUM DIOXIDE (CI 77891), TIN OXIDE], Sensiva^{®} PA 40 [INCI: PHENYLPROPANOL, PROPANEDIOL, CAPRYLYL GLYCOL, TOCOPHEROL], Zemea^{®} [INCI: PROPANEDIOL], is

premixed and added to the previous mixture under stirring and mixed until homogeneous.

Phase D: Ethyl Alcohol Denat. [INCI: ALCOHOL DENAT.], Fragrance [INCI: FRAGANCE (PARFUM)], is added and mixed with moderate stirring until homogeneous.

Phase E: Ligustrum lucidum subcritical water extract according to Example 2 [INCI: GLYCERIN, LIGUSTRUM LUCIDUM SEED EXTRACT] is added to the previous mixture.

Phase F: Avalure^{™} UR 450 polymer [INCI: WATER (AQUA), PPG-17/IPDI/DMPA COPOLYMER], Dissolvine^{®} NA2-P [DISODIUM EDTA], is premixed and added to the previous mixture under stirring until homogeneous.

**Table 13**

| Phase | INGREDIENT (INCI name) | % weight |
|---|---|---|
| A | WATER (AQUA) | 78.85 |
| A | [WATER (AQUA), ACRYLATES COPOLYMER] | 3.0 |
| A | [WATER (AQUA), ACRYLATES/BEHENETH-25 METHACRYLATE COPOLYMER] | 0.5 |
| B | [WATER (AQUA), SODIUM HYDROXIDE] | 0.6 |
| C | [TITANIUM DIOXIDE (CI 77891), MICA, TIN OXIDE] | 0.5 |
| C | [TITANIUM DIOXIDE (CI 77891), MICA, TIN OXIDE] | 0.5 |
| C | [MICA, TITANIUM DIOXIDE (CI 77891), IRON OXIDES (Cl 77491)] | 0.1 |
| C | [CALCIUM ALUMINUM BOROSILICATE, SILICA, TITANIUM DIOXIDE (CI 77891), TIN OXIDE] | 0.1 |
| C | [PHENYLPROPANOL, PROPANEDIOL, CAPRYLYL GLYCOL, TOCOPHEROL] | 0.8 |
| C | PROPANEDIOL | 5.0 |
| D | ALCOHOL DENAT. | 5.0 |
| D | FRAGRANCE (PARFUM) | 0.05 |
| E | [GLYCERIN, LIGUSTRUM LUCIDUM SEED EXTRACT] | 2.0 |
| F | [WATER (AQUA), PPG-17/IPDI/DMPA COPOLYMER] | 3.0 |

### EXAMPLE 17

### Lotion comprising Ligustrum lucidum subcritical water extract

In a suitable vessel, the ingredients of phase A1 are weighted: water [INCI: WATER (AQUA)], Zemea^{®} [INCI: PROPANEDIOL], glycerin [INCI: GLYCERIN], potassium sorbate [INCI: POTASSIUM SORBATE] and Dissolvine^{®} NA2 [INCI: DISODIUM EDTA] are dissolved.

Phase A2 ingredient: Carbopol^{®} Ultrez 30 Polymer [INCI: CARBOMER] is added in the previous mixture. Once dispersed, phase A3: xanthan gum [INCI: XANTHAN GUM] is introduced. Then the mixture is heated at 70-75°C.

In a separate vessel, phase B ingredients: Fancor^{®} Meadowfoam seed oil [INCI: LIMNANTHES ALBA (MEADOWFOAM) SEED OIL], Kodasil^{™} 600 IDD Gel [INCI: ISODODECANE; VINYL DIMETHICONE/LAURYL DIMETHICONE CROSSPOLYMER; DIMETHICONE; LAURYL DIMETHICONE], Astro-sil 2C 350 [INCI: DIMETHICONE], Schercemol^{™} CATC ester [INCI: COCOYL ADIPIC ACID/TRIMETHYLOLPROPANE COPOLYMER; TRIMETHYLOLPROPANE], Schercemol^{™} DIS ester [INCI: DIISOPROPYL SEBACATE], Tocopheryl Acetate [INCI: TOCOPHERYL ACETATE] and Phenoxetol^{™} [INCI: PHENOXYETHANOL] are mixed and the resulting mixture is heated at 70-75°C.

The emulsion is made by adding slowly phase B onto phase A under fast stirring with a turbine.

Once the mixture is cooled to 40°C, phase C: Novemer^{™} EC-2 polymer [INCI: WATER (AQUA); SODIUM ACRYLATES/BEHENETH-25 METHACRYLATE CROSSPOLYMER; HYDROGENATED POLYDECENE, LAURYL GLUCOSIDE], SA-SB-300 (7%) [INCI: SILICA; DIMETHICONE], Fragrance [INCI: FRAGANCE (PARFUM)], and Ligustrum lucidum subcritical water extract according to Example 2 [INCI: GLYCERIN, LIGUSTRUM LUCIDUM SEED EXTRACT], are added to the previous mixture.

pH is adjusted to 6.0 - 6.5 with phase D ingredient sodium hydroxide 20% w/w [INCI: WATER (AQUA); SODIUM HYDROXIDE]).

**Table 14**

| Phase | INGREDIENT (INCI name) | % weight |
|---|---|---|
| A1 | WATER | 63.60 |
| A1 | PROPANEDIOL | 10.00 |
| A1 | GLYCERIN | 5.00 |
| A1 | POTASSIUM SORBATE | 0.10 |
| A1 | DISODIUM EDTA | 0.20 |
| A2 | CARBOMER | 0.30 |
| A3 | XANTHAN GUM | 0.20 |
| B | LIMNANTHES ALBA (MEADOWFOAM) SEED OIL | 5.00 |
| B | [ISODODECANE; VINYL DIMETHICONE/LAURYL DIMETHICONE CROSSPOLYMER; DIMETHICONE; LAURYL DIMETHICONE] | 3.00 |
| B | DIMETHICONE | 3.00 |
| B | [COCOYL ADIPIC ACID/TRIMETHYLOLPROPANE COPOLYMER; TRIMETHYLOLPROPANE] | 2.00 |
| B | DIISOPROPYL SEBACATE | 2.00 |
| B | TOCOPHERYL ACETATE | 0.50 |
| B | PHENOXYETHANOL | 0.50 |
| C | [WATER (AQUA); SODIUM ACRYLATES/BEHENETH-25 METHACRYLATE CROSSPOLYMER; HYDROGENATED POLYDECENE, LAURYL GLUCOSIDE] | 1.50 |
| C | [SILICA; DIMETHICONE] | 1.00 |
| C | [GLYCERIN, LIGUSTRUM LUCIDUM SEED EXTRACT] | 2.00 |
| C | FRAGANCE (PARFUM) | 0.10 |
| D | [WATER (AQUA); SODIUM HYDROXIDE] | q.s. |

### EXAMPLE 18

### Facial mask comprising Ligustrum lucidum subcritical water extract

In a suitable vessel, the ingredients of phase A: water [INCI: WATER (AQUA)], Carbopol^{®} Ultrez 30 polymer [INCI: CARBOMER], are weighted and placed under moderate stirring until complete dispersion.

Phase B ingredient: GENENCARE^{®} OSMS BA [INCI: BETAINE] is added in the previous mixture and mixed until homogeneous.

Phase C ingredients: xanthan gum [INCI: XANTHAN GUM], Zemea^{®} [INCI: PROPANEDIOL], glycerin [INCI: GLYCERIN], are premixed in a separate vessel and introduced to the previous mixture.

Phase D: Sensiva^{®} PA 40 [INCI: PHENYLPROPANOL, PROPANEDIOL, CAPRYLYL GLYCOL, TOCOPHEROL], Phase E: Hydramol^{™} TGL ester [INCI: POLYGLYCERYL-3 LAURATE] and Phase F: subcritical water extract [INCI: GLYCERIN, LIGUSTRUM LUCIDUM SEED EXTRACT], are added one-by-one under moderate stirring and mixed until homogeneous.

pH is adjusted to 4.5 - 5.0 with phase G ingredient sodium hydroxide 20% w/w [INCI: WATER (AQUA); SODIUM HYDROXIDE])

Phase H: Chembetaine^{™} CAD surfactant [INCI: WATER (AQUA), COCAMIDOPROPYL BETAINE, SODIUM CHLORIDE, TETRASODIUM EDTA] is added with slow stirring and mixed until homogeneous.

Finally, phase I: Belsil^{®} DM 0.65 [INCI: DISILOXANE] is added with slow stirring and mixed until homogeneous.

**Table 15**

| Phase | INGREDIENT (INCI name) | % weight |
|---|---|---|
| A | WATER (AQUA) | 60.6 |
| A | CARBOMER | 0.8 |
| B | BETAINE | 4.0 |
| C | XANTHAN GUM | 0.3 |
| C | PROPANEDIOL | 10.0 |
| C | GLYCERIN | 5.0 |
| D | [PHENYLPROPANOL, PROPANEDIOL, CAPRYLYL GLYCOL, TOCOPHEROL] | 0.8 |
| E | POLYGLYCERYL-3 LAURATE | 1.0 |
| F | [GLYCERIN, LIGUSTRUM LUCIDUM SEED EXTRACT] | 2.0 |
| G | [WATER (AQUA), SODIUM HYDROXIDE] | 0.5 |
| H | [WATER (AQUA), COCAMIDOPROPYL BETAINE, SODIUM CHLORIDE, TETRASODIUM EDTA] | 10.0 |
| I | DISILOXANE | 5.0 |

### EXAMPLE 19

### Serum comprising Ligustrum lucidum subcritical water extract

In a suitable vessel, the ingredients of phase A: water [INCI: WATER (AQUA)], Pemulen^{™} EZ-4U polymeric emulsifier [INCI: ACRYLATES/ C10/30 ALKYL ACRYLATE CROSSPOLYMER] are weighted and mixed until complete dispersion.

Phase B ingredients: Kelco-Care^{™} Diutan Gum [INCI: SPHINGOMONAS FERMENT EXTRACT], Zemea^{®} [INCI: PROPANEDIOL], are premixed in a separate vessel and introduced to the previous mixture under moderate stirring.

Ingredients of phase C: dermosoft^{®} 700B [INCI: LEVULINIC ACID, SODIUM LEVULINATE, WATER (AQUA), GLYCERIN], Hydramol^{™} TGL ester [INCI: POLYGLYCERYL-3 LAURATE] are added one-by-one under moderate stirring and mixed until homogeneous.

The mixture is heated up to 35-40°C and phase D: dermosoft^{®} GMCY MB [INCI: GLYCERYL CAPRYLATE] is added under moderate mixing.

Phase E: AlgaPu̅r^{™} High Stability High Oleic (HSHO) Algae Oil [INCI: TRIOLEIN, TOCOPHEROL, HELIANTHUS ANNUUS SEED OIL] is added to the previous mixture under stirring until homogeneous.

Phase F: Ligustrum lucidum subcritical water extract according to Example 2 [INCI: GLYCERIN, LIGUSTRUM LUCIDUM SEED EXTRACT] is added to the previous mixture under stirring until homogeneous.

Phase G ingredients: L-Arginine [INCI: ARGININE], water [INCI: WATER (AQUA)], are premixed in a separate vessel and introduced to the previous mixture under stirring until homogeneous.

Finally, phase H: Timiron^{®} SynBeam Violet [INCI: SYNTHETIC FLUORPHLOGOPITE, TITANIUM DIOXIDE (CI 77891), TIN OXIDE], water [INCI: WATER (AQUA)], are premixed in a separate vessel and introduced to the previous mixture under stirring until homogeneous.

**Table 16**

| Phase | INGREDIENT (INCI name) | % weight |
|---|---|---|
| A | WATER (AQUA) | 82.91 |
| A | ACRYLATES/C10-30 ALKYL ACRYLATE CROSSPOLYMER | 0.3 |
| B | SPHINGOMONAS FERMENT EXTRACT | 0.29 |
| B | PROPANEDIOL | 5.0 |
| C | [LEVULINIC ACID, SODIUM LEVULINATE, WATER (AQUA), GLYCERIN] | 1.0 |
| C | POLYGLYCERYL-3 LAURATE | 0.5 |
| D | GLYCERYL CAPRYLATE | 1.0 |
| E | [TRIOLEIN, TOCOPHEROL, HELIANTHUS ANNUUS SEED OIL] | 2.0 |
| F | [GLYCERIN, LIGUSTRUM LUCIDUM SEED EXTRACT] | 2.0 |
| G | ARGININE | 0.5 |
| G | WATER (AQUA) | 2.0 |
| H | [SYNTHETIC FLUORPHLOGOPITE, TITANIUM DIOXIDE (CI 77891), TIN OXIDE] | 0.5 |
| H | WATER (AQUA) | 2.0 |

### EXAMPLE 20

### In vivo study for the assessment of the skin oxygenation effect of the botanical extract of the invention after long-term application in Caucasian skin type female volunteers.

The study was carried out for 28 days. Eighty-four (84) Caucasian female volunteers, aged between 25 and 55 years old were included in the study and were subdivided into three groups. Thirty (30) subjects applied the composition described in Example 9 (Active Cream) on whole face and twenty-eight (28) subjects applied a Placebo Cream having the same composition except that the botanical extract of the invention was not present. Active and Placebo Creams were applied for 28 days twice a day (morning and evening). Subjects served as their own reference and results obtained at time 28 days were compared with those obtained at initial time. Moreover, results obtained with the Active Cream were compared with those obtained with Placebo Cream. The third group of subjects, which included 26 volunteers, performed a yoga session. No Active or Placebo Cream was applied to these subjects and post-yoga session results are compared with those obtained at initial time.

*Skin oxygenation:* TiVi oxygen mapper provides a measurement of skin oxygenation by measuring the diffuse back-scattered light in the visible region of volunteer's skin. With this parameter, the tool measures the presence of oxyhemoglobin in capillary blood vessels of volunteers. Measurements were taken at initial time for the three groups. Endpoint measurement was done at day 28 for Active and Placebo Creams and post-Yoga session for Yoga session subjects. Results are shown in below:

**Table 17. Skin oxygenation increase after 28 days of product application. Statistical significance respect initial time**

| | Mean change of oxyhemoglobin at endpoint |
|---|---|
| Active Cream | 22.79 |
| Placebo Cream | -11.13 |
| Yoga session | 19.42 |

The results demonstrate that, after 28 days of application of the composition of the invention, there is a statistically significant increase of skin oxygenation compared to initial time. Moreover, the increase of skin oxygenation is higher with the Active Cream than with a Yoga session.

*Skin luminosity* (parameter L*): measured in macrophotographies using cross-polarized filters. All groups initial measurements were taken at initial time and endpoint measurement were done at day 28 for Active and Placebo Cream and post-Yoga session for Yoga session subjects.

**Table 18. Parameter L* before and after 28 days of product application. Statistical significance respect initial time: *p<0.05 calculated using an unpaired Student's t test vs initial time**

| | Mean increase of L* (%) |
|---|---|
| Active Cream | 1.28 * |
| Placebo Cream | -0.22 |
| Yoga session | -0.19 |

The results demonstrate that, after 28 days of application of the composition of the invention, there is a significant increase of luminosity of skin compared to Placebo or Yoga session.

*Crow's feet area wrinkle depth:* Images of volunteer's crow's feet were taken with a 3D microtopography imaging system. Wrinkle depth measurements were done at initial time and after 28 days of product application or post-Yoga session.

**Table 19. Crow's feet area wrinkle depth decreases after 28 days of product application or post-Yoga session**

| | Mean increase of wrinkle depth (%) |
|---|---|
| Active Cream | -6.39 |
| Placebo Cream | 7.29 |
| Yoga session | -7.09 |

The results demonstrate that, after 28 days of application of the composition of the invention, there is a statistically significant decrease of crow's feet area wrinkle depth compared to initial time. Moreover, the decrease in wrinkle depth is higher with the Active Cream than with Placebo Cream and comparable to the achieved in a Yoga session.

*Crow's feet area wrinkle volume:* Images of volunteer's crow's feet area were taken with a 3D microtopography imaging system. Wrinkle volume measurements were carried out at initial time and after 28 days of product application or post-Yoga session.

**Table 20. Crow's feet area wrinkle volume decreases after 28 days of product application or post-Yoga session. Statistical significance respect initial time**

| | Mean increase of wrinkle volume (%) |
|---|---|
| Active Cream | -15.81 |
| Placebo Cream | -5.08 |
| Yoga session | -8.84 |

The results demonstrate that, after 28 days of product application, there is a statistically significant decrease of crow's feet wrinkle volume compared to initial time. Moreover, the decrease in wrinkle volume is higher with the Active Cream than the Placebo Cream and the Yoga session.

*Underneath eye area wrinkle depth:* Images of volunteer's underneath eye area were taken with a 3D microtopography imaging system. Wrinkle depth measurements were done at initial time and after 28 days of product application or post-Yoga session.

**Table 21. Underneath eye area wrinkle depth decrease after 28 days of product application or post-Yoga session. Statistical significance respect initial time**

| | Mean increase of wrinkle depth (%) |
|---|---|
| Active Cream | -6.39 |
| Placebo Cream | -0.13 |
| Yoga session | -1.81 |

The results demonstrate that, after 28 days of product application, there is a statistically significant decrease of wrinkle depth compared to initial time. Moreover, the decrease in wrinkle depth is higher with the active cream than the placebo or a yoga session.

*Underneath eye area skin roughness:* Images of the area underneath volunteer's eyes were taken with a 3D microtopography imaging system. Parameter "Sa", defined as the arithmetic mean of the surface roughness (µm) was measured at initial time and after 28 days of product application or post-Yoga session.

**Table 22. Underneath eye area skin roughness decrease after 28 days of product application or post-Yoga session**

| | Mean increase of wrinkle depth (%) |
|---|---|
| Active Cream | -5.62 |
| Placebo Cream | 0.42 |
| Yoga session | -3.88 |

Results demonstrate that, after 28 days of product application, there is a statistically significant decrease of skin roughness compared to initial time. Moreover, skin roughness reduction is higher with the Active Cream than the achieved with the Placebo Cream or after a Yoga session.

*Self-questionnaire:* after 28 days of Active Cream and Placebo Cream, the volunteers answered a self-questionnaire to evaluate the efficacy of both products.

**Table 23. Percentage of positive responses to skin-related questions after 28 days of product application**

| | % of positive responses Active cream | % of positive responses Placebo cream |
|---|---|---|
| Would you buy/recommend the product | 83.87 | 81.48 |
| Keep on using the product | 90.32 | 88.89 |
| After the use of the product I feel more beautiful | 64.52 | 55.55 |
| After the use of the product my appearance is healthier | 83.87 | 88.89 |
| The product reduces fine lines | 51.62 | 62.97 |
| After the use of the product I have noticed a reduction of fine lines | 45.16 | 40.74 |
| The product improves my skin luminosity | 87.09 | 81.48 |
| The product improves my skin softness | 87.10 | 85.19 |
| The product improves the pink tone of my skin | 70.96 | 55.55 |
| The product helps to mimic the pink and luminous tone obtained | 51.62 | 55.55 |
| after a Yoga session | | |

The results demonstrate that, after 28 days, the Active Ceam showed a higher improvement than the Placebo Cream, according to the volunteers' answers.

### EXAMPLE 21

### Large scale subcritical water extract from Liqustrum lucidum

The extract was obtained by a process comprising a water extraction in the subcritical state in dynamic mode following the steps of:
i) Grinding the *L. lucidum* fruitto obtain particles with size corresponding to from about 0.5 mm to about 1 mm mesh.
ii) Loading 120g of the crushed *Ligustrum lucidum* into a 300mL reactor, which is placed in an oven previously heated to a temperature above 100°C, and then water was pumped through the reactor for 220 min.
iii) Passing 3300mL of water (1:27 ratio of raw material:water) in the subcritical state through the plant material, at a flow rate of 15 mL/min, a water temperature of 160°C and a pressure of 1.5 MPathe aqueous extract was obtained.
iv) Mixing part of the aqueous extract obtained was with 100% glycerin by weight to make a 80% glycerin by weight solution and thus obtain a final concentration of raw material in 80% glycerin solution of 1:100.

### EXAMPLE 22

Subcritical water extract from Ligustrum lucidum obtained at different temperatures

Subcritical water extracts were obtained as described in Example 1 but performing the extraction at 140°C, 150°C, 170°C or 180°C.

**Table 24. Quantified bio-actives in Ligustrum lucidum obtained by different SW conditions. ND= not detected**

| | SWE 140°C | SWE 150°C | SWE 170°C | SWE 180°C |
|---|---|---|---|---|
| vanillin | 0.4979 | 0.4996 | 0.4816 | 0.7448 |
| Luteolin-7-O-glucoside | 6.9825 | 6.5671 | 4.3366 | 5.0712 |
| 3,4/3,5-dihydroxybenzoic acid | 0.4144 | 0.5516 | 0.6458 | 1.0348 |
| 4-hydroxybenzaldehyde | 0.1257 | 0.1450 | 0.1347 | 0.1760 |
| 4-Hydroxybenzoic acid | 1.3270 | 1.5026 | 1.0980 | 1.0339 |
| vanillic acid | 0.3696 | 0.3850 | 0.3632 | 0.4993 |
| caffeic acid | 0.2850 | 0.3960 | 0.4014 | 0.3376 |
| ferulic acid | 0.1111 | 0.1308 | 0.1120 | 0.1028 |
| Abscisic acid | 0.0098 | 0.0119 | 0.0072 | 0.0072 |
| Quercetin | 0.1445 | 0.1329 | 0.0781 | 0.1032 |
| Eriodictyol | 0.0201 | 0.0108 | 0.0082 | 0.0148 |
| Sinapyl-alcohol | 0.1552 | 0.1333 | 0.0998 | 0.1087 |
| Salicylic acid | 0.1017 | 0.0980 | 0.0924 | 0.1620 |
| Coniferaldehyde | 0.1601 | 0.2442 | 0.2443 | 0.6086 |
| Apigenin-7-O-glucoside | 0.2201 | 0.1921 | 0.1340 | 0.1879 |
| Cinnamic acid | 0.6402 | 0.6727 | 0.6117 | 1.2171 |
| Coniferyl-alcohol | 3.0936 | 8.1302 | 9.7267 | 10.9713 |
| Coumaric acid | 0.5436 | 0.4381 | 0.2989 | 0.4156 |
| Myricetin | 0.4283 | 0.4980 | 0.3902 | 0.9048 |
| Kaempferol | 0.2752 | 0.1741 | 0.0930 | 0.0646 |
| 3,5-dihydroxybenzaldehyde | 0.1355 | 0.2058 | 0.2058 | 0.0877 |
| Kaempferol-3-O-glucoside | ND | ND | ND | ND |
| Rutin | ND | ND | ND | ND |
| Luteolin | 0.0101 | 0.0128 | 0.0070 | 0.0130 |
| 3,4 & 3,5-dihydroxybenzoic acid (co-ellute) | ND | ND | ND | ND |
| 2,5-dihydroxybenzoic acid | ND | ND | ND | ND |
| alpha-Amyrin | ND | ND | ND | ND |
| Naringenin | 0.0264 | 0.0378 | 0.0314 | 0.0556 |
| Apigenin | 3.3255 | 3.2305 | 2.2091 | 2.8487 |
| Oleuropein | 23.1482 | 16.1489 | 5.9794 | 1.6284 |
| Salidroside | 26.4376 | 52.8648 | 56.2853 | 156.1482 |
| Total phenolics | 68.9889 | 93.4147 | 84.0755 | 184.5478 |

All the extracts contained under this range of temperatures comprise coniferyl-alcohol, myricetin, apigenin, oleuropein and salidroside.

In contrast, none of the extracts obtained in Examples 4 to 8 comprised myricetin, and did not comprise coniferyl-alcohol, myricetin, apigenin, oleuropein and salidroside.

## Claims

1. A process for obtaining a botanical extract from fruit of *Ligustrum lucidum* comprising subjecting fruit of *Ligustrum lucidum* to an extraction with subcritical water, wherein the extraction comprises a step of contacting the fruit with subcritical water at a temperature of
from 140°C to 180°C, or from 150°C to 175°C, or from 155°C to 170°C, or from 155°C to 165°C, or from 160°C to 165°C,
and at a pressure suitable to maintain the water in a liquid state for a period of at least 5 minutes to form an aqueous botanical extract.

2. A process according to claim 1, wherein the fruit is dried fruit and the ratio of the weight of dried fruit to the volume of subcritical water is from 1:5 to 1:50 g/mL (w/v), preferably wherein the fruit is in the form of particles.

3. A process according to claim 1 or claim 2, wherein the step of contacting the fruit with subcritical water is carried out for a period of at least 15 minutes, at least 30 minutes, at least 1 hour, at least 2 hours or at least about 3 hours, and/or wherein the subcritical water is at a pressure of from 0.5 to 20 MPa, or from 1 MPa to 2 MPa.

4. A process according to any one of the preceding claims, wherein the extraction is with subcritical water only.

5. A process according to anyone of the preceding claims, wherein the extraction with subcritical water is performed in a dynamic mode and comprises contacting the fruit with a continuous flow of the subcritical water, wherein the subcritical water has a flow rate of from 5 to 30 mL/min.

6. A process according to any one of the preceding claims, comprising a step of separating the fruit from the aqueous botanical extract and comprising a step of purifying the aqueous botanical extract to obtain a purified aqueous botanical extract, wherein purifying the aqueous botanical extract comprises concentrating the aqueous botanical extract to obtain a concentrate of the aqueous botanical extract, preferably drying the aqueous botanical extract to obtain a solid form of the botanical extract.

7. An aqueous botanical extract or a purified aqueous botanical extract, wherein the respective extract is obtainable by the process of any one of claims 1 to 6, comprising oleuropein, salidroside and myricetin.

8. An aqueous botanical extract or a purified aqueous botanical extract according to claim 7, further comprising coniferyl alcohol and/or apigenin, preferably further comprising at least one compound selected from vanillin, 3,4-dihydroxybenzoic acid, 3,5-dihydroxybenzoic acid, 4-hydroxybenzaldehyde, 4-hydroxybenzoic acid, caffeic acid, quercetin, sinapyl-alcohol, salicylic acid, kaempferol and naringenin.

9. A composition comprising the aqueous botanical extract or the purified aqueous botanical extract of claim 7 or claim 8, and a water-miscible organic solvent, wherein the water-miscible organic solvent is a polyol.

10. A composition according to claim 9, wherein the polyol is present in an amount of from 50 to 90 wt.% based on the weight of the total composition.

11. A composition according to claim 9 or claim 10, comprising from 1 to 40 ppm oleuropein, from 1 to 40 ppm salidroside and from 0.1 to 3 ppm myricetin, and preferably, from 1 or 2 to 25 ppm coniferyl-alcohol (4-[(E)-3-hydroxyprop-1-enyl]-2-methoxyphenol) and/or from 0.01 to 5 ppm apigenin.

12. A cosmetic composition comprising: the aqueous botanical extract or the purified aqueous botanical extract of claim 7 or claim 8, or the composition of any one of claims 9 to 11; and at least one cosmetically acceptable excipient or ingredient.

13. Use of an aqueous botanical extract or a purified aqueous botanical extract according to claim 7 or claim 8 for improving or increasing skin microcirculation and/or vascularization, and/or improving or increasing skin oxygenation.

14. Use of an aqueous botanical extract or a purified aqueous botanical extract according to claim 7 or claim 8 for improving skin rosiness (natural flush); reducing and/or preventing the symptoms of skin aging; increasing skin smoothness; and/or improving or increasing skin glossiness and/or luminosity.

15. A method for the cosmetic, non-therapeutic treatment and/or care of the skin comprising topically administering an aqueous botanical extract or a purified aqueous botanical extract according to claim 7 or claim 8, or a composition according to any one of claims 9 to 12 to the skin, wherein the cosmetic non-therapeutic treatment and/or care of the skin is: the improvement and/or increase of skin microcirculation or vascularization; the improvement or increase of skin oxygenation; the improvement or increase of skin rosiness (natural flush); the reduction and/or prevention of the symptoms of skin aging; the increase of skin smoothness; and/or the improvement or increase of skin glossiness and/or luminosity.

## Patentansprüche

1. Prozess zum Erhalten eines Pflanzenextrakts aus einer Frucht von *Ligustrum lucidum*, umfassend ein Unterziehen der Frucht von *Ligustrum lucidum* einer Extraktion mit subkritischem Wasser, wobei die Extraktion einen Schritt eines Inberührungbringens der Frucht mit subkritischem Wasser bei einer Temperatur umfasst
von 140 °C bis 180 °C oder von 150 °C bis 175 °C oder von 155 °C bis 170 °C oder von 155 °C bis 165 °C oder von 160 °C bis 165 °C
und bei einem Druck, der geeignet ist, um das Wasser für einen Zeitraum von mindestens 5 Minuten in einem flüssigen Zustand zu halten, um einen wässrigen Pflanzenextrakt auszubilden.

2. Prozess nach Anspruch 1, wobei die Frucht eine getrocknete Frucht ist und das Verhältnis des Gewichts der getrockneten Frucht zu dem Volumen des subkritischen Wassers von 1 : 5 bis 1 : 50 g/ml (w/v) beträgt, vorzugsweise wobei die Frucht in der Form von Teilchen vorliegt.

3. Prozess nach Anspruch 1 oder 2, wobei der Schritt des Inberührungbringens der Frucht mit subkritischem Wasser für einen Zeitraum von mindestens 15 Minuten, mindestens 30 Minuten, mindestens 1 Stunde, mindestens 2 Stunden oder mindestens etwa 3 Stunden ausgeführt wird und/oder wobei das subkritische Wasser bei einem Druck von 0,5 bis 20 MPa oder von 1 MPa bis 2 MPa vorliegt.

4. Prozess nach einem der vorstehenden Ansprüche, wobei die Extraktion nur mit subkritischem Wasser erfolgt.

5. Prozess nach einem der vorstehenden Ansprüche, wobei die Extraktion mit subkritischem Wasser in einem dynamischen Modus durchgeführt wird und das Inberührungbringen der Frucht mit einer kontinuierlichen Strömung des subkritischen Wassers umfasst, wobei das subkritische Wasser eine Strömungsgeschwindigkeit von 5 bis 30 ml/min aufweist.

6. Prozess nach einem der vorstehenden Ansprüche, umfassend einen Schritt eines Trennens der Frucht von dem wässrigen Pflanzenextrakt und umfassend einen Schritt eines Reinigens des wässrigen Pflanzenextrakts, um einen gereinigten wässrigen Pflanzenextrakt zu erhalten, wobei das Reinigen des wässrigen Pflanzenextrakts ein Konzentrieren des wässrigen Pflanzenextrakts umfasst, um ein Konzentrat des wässrigen Pflanzenextrakts zu erhalten, vorzugsweise ein Trocknen des wässrigen Pflanzenextrakts, um eine feste Form des Pflanzenextrakts zu erhalten.

7. Wässriger Pflanzenextrakt oder gereinigter wässriger Pflanzenextrakt, wobei der jeweilige Extrakt durch den Prozess nach einem der Ansprüche 1 bis 6 erhältlich ist, umfassend Oleuropein, Salidrosid und Myricetin.

8. Wässriger Pflanzenextrakt oder gereinigter wässriger Pflanzenextrakt nach Anspruch 7, ferner umfassend Coniferylalkohol und/oder Apigenin, vorzugsweise ferner umfassend mindestens eine Verbindung, die aus Vanillin, 3,4-Dihydroxybenzoesäure, 3,5-Dihydroxybenzoesäure, 4-Hydroxybenzaldehyd, 4-Hydroxybenzoesäure, Kaffeesäure, Quercetin, Sinapylalkohol, Salicylsäure, Kaempferol und Naringenin ausgewählt ist.

9. Zusammensetzung, umfassend den wässrigen Pflanzenextrakt oder den gereinigten wässrigen Pflanzenextrakt nach Anspruch 7 oder 8 und ein wassermischbares organisches Lösungsmittel, wobei das wassermischbare organische Lösungsmittel ein Polyol ist.

10. Zusammensetzung nach Anspruch 9, wobei das Polyol in einer Menge von 50 bis 90 Gew.-%, basierend auf dem Gewicht der Gesamtzusammensetzung, vorhanden ist.

11. Zusammensetzung nach Anspruch 9 oder 10, umfassend von 1 bis 40 ppm Oleuropein, von 1 bis 40 ppm Salidrosid und von 0,1 bis 3 ppm Myricetin und vorzugsweise von 1 oder 2 bis 25 ppm Coniferylalkohol (4-[(E)-3-Hydroxyprop-1-enyl]-2-methoxyphenol) und/oder von 0,01 bis 5 ppm Apigenin.

12. Kosmetische Zusammensetzung, umfassend: den wässrigen Pflanzenextrakt oder den gereinigten wässrigen Pflanzenextrakt nach Anspruch 7 oder 8 oder die Zusammensetzung nach einem der Ansprüche 9 bis 11; und mindestens einen kosmetisch verträglichen Arzneistoffträger oder Inhaltsstoff.

13. Verwendung eines wässrigen Pflanzenextrakts oder eines gereinigten wässrigen Pflanzenextrakts nach Anspruch 7 oder 8 zum Verbessern oder Erhöhen einer Mikrozirkulation und/oder einer Vaskularisation der Haut und/oder Verbessern oder Erhöhen einer Oxygenierung der Haut.

14. Verwendung eines wässrigen Pflanzenextrakts oder eines gereinigten wässrigen Pflanzenextrakts nach Anspruch 7 oder 8 zum Verbessern einer Rosigkeit der Haut (natürliche Rötung); Verringern und/oder Vorbeugen der Symptome von Hautalterung; Erhöhen einer Glätte der Haut; und/oder Verbessern oder Erhöhen eines Glanzes und/oder einer Leuchtkraft der Haut.

15. Verfahren für die kosmetische, nicht therapeutische Behandlung und/oder Pflege der Haut, umfassend ein topisches Verabreichen eines wässrigen Pflanzenextrakts oder eines gereinigten wässrigen Pflanzenextrakts nach Anspruch 7 oder 8 oder einer Zusammensetzung nach einem der Ansprüche 9 bis 12 auf die Haut, wobei die kosmetische, nicht therapeutische Behandlung und/oder Pflege der Haut ist: die Verbesserung und/oder die Erhöhung der Mikrozirkulation oder Vaskularisation der Haut; die Verbesserung oder die Erhöhung der Oxygenierung der Haut; die Verbesserung oder die Erhöhung der Rosigkeit der Haut (natürliche Rötung); die Verringerung und/oder die Vorbeugung der Symptome von Hautalterung; die Erhöhung der Glätte der Haut; und/oder die Verbesserung oder die Erhöhung des Glanzes und/oder der Leuchtkraft der Haut.

## Revendications

1. Procédé d'obtention d'un extrait botanique à partir d'un fruit de *Ligustrum lucidum* comprenant la soumission d'un fruit de *Ligustrum lucidum* à une extraction avec de l'eau subcritique, dans lequel l'extraction comprend une étape de mise en contact du fruit avec de l'eau subcritique à une température
de 140 °C à 180 °C, ou de 150 °C à 175 °C, ou de 155 °C à 170 °C, ou de 155 °C à 165 °C, ou de 160 °C à 165 °C,
et à une pression appropriée pour maintenir l'eau à l'état liquide pendant une période d'au moins 5 minutes pour former un extrait botanique aqueux.

2. Procédé selon la revendication 1, dans lequel le fruit est un fruit sec et le rapport entre le poids du fruit sec et le volume d'eau sous-critique est de 1:5 à 1:50 g/mL (p/v), de préférence dans lequel le fruit se présente sous la forme de particules.

3. Procédé selon la revendication 1 ou la revendication 2, dans lequel l'étape consistant à mettre en contact le fruit avec de l'eau sous-critique est effectuée pendant un laps de temps d'au moins 15 minutes, au moins 30 minutes, au moins 1 heure, au moins 2 heures ou au moins environ 3 heures, et/ou dans lequel l'eau sous-critique est à une pression comprise entre 0,5 et 20 MPa, ou de 1 MPa à 2 MPa.

4. Procédé selon l'une quelconque des revendications précédentes, dans lequel l'extraction se fait uniquement avec de l'eau sous-critique.

5. Procédé selon l'une quelconque des revendications précédentes, dans lequel l'extraction avec de l'eau sous-critique est mise en œuvre dans un mode dynamique et comprend la mise en contact du fruit avec un écoulement continu de l'eau sous-critique, dans lequel l'eau sous-critique a un débit allant de 5 à 30 mL/min.

6. Procédé selon l'une quelconque des revendications précédentes, comprenant une étape de séparation du fruit de l'extrait botanique aqueux et comprenant une étape de purification de l'extrait botanique aqueux pour obtenir un extrait botanique aqueux purifié, dans lequel la purification de l'extrait botanique aqueux comprend la concentration de l'extrait botanique aqueux pour obtenir un concentré de l'extrait botanique aqueux, de préférence le séchage de l'extrait botanique aqueux pour obtenir une forme solide de l'extrait botanique.

7. Extrait botanique aqueux ou extrait botanique aqueux purifié, dans lequel l'extrait respectif peut être obtenu par le procédé selon l'une quelconque des revendications 1 à 6, comprenant de l'oleuropéine, du salidroside et de la myricétine.

8. Extracteur botanique aqueux, extrait botanique aqueux purifié selon la revendication 7, comprenant en outre de l'alcool conifère et/ou de l'apigénine, comprenant de préférence en outre au moins un composé choisi parmi vanilline, acide 3,4-dihydroxybenzoïque, acide 3,5-dihydroxybenzoïque, 4-hydroxybenzaldéhyde, acide 4-hydroxybenzoïque, acide caféique, quercétine, alcool sinapylique, acide salicylique, kaempférol et naringénine.

9. Composition comprenant l'extrait botanique aqueux ou l'extrait botanique aqueux purifié selon la revendication 7 ou la revendication 8, et un solvant organique miscible à l'eau, dans laquelle le solvant organique miscible à l'eau est un polyol.

10. Composition selon la revendication 9, dans laquelle le polyol est présent en une quantité allant de 50 à 90 % en poids sur la base du poids de la composition totale.

11. Composition selon la revendication 9 ou la revendication 10, comprenant de 1 à 40 ppm d'oleuropéine, de 1 à 40 ppm de salidroside et de 0,1 à 3 ppm myricétine, et de préférence de 1 ou 2 à 25 ppm d'alcool conifère (4-[(E)-3-hydroxyprop-1-ényl]-2-méthoxyphénol) et/ou de 0,01 à 5 ppm apigénine.

12. Composition cosmétique comprenant : l'extrait botanique aqueux ou l'extrait botanique aqueux purifié selon la revendication 7 ou la revendication 8, ou la composition selon l'une quelconque des revendications 9 à 11 ; et au moins un excipient ou ingrédient cosmétiquement acceptable.

13. Utilisation d'un extrait botanique aqueux ou d'un extrait botanique aqueux purifié selon la revendication 7 ou la revendication 8 pour améliorer ou augmenter la microcirculation cutanée et/ou la vascularisation, et/ou améliorer ou augmenter l'oxygénation de la peau.

14. Utilisation d'un extrait botanique aqueux ou d'un extrait botanique aqueux purifié selon la revendication 7 ou la revendication 8 pour améliorer la rosie de la peau (rougeur naturelle) ; réduire et/ou prévenir les symptômes du vieillissement cutané ; augmenter la douceur de la peau ; et/ou améliorer ou augmenter la brillance et/ou la luminosité de la peau.

15. Procédé cosmétique, non thérapeutique de traitement et/ou de soin de la peau comprenant l'administration topique d'un extrait botanique aqueux ou d'un extrait botanique aqueux purifié selon la revendication 7 ou de la revendication 8, ou d'une composition selon l'une quelconque des revendications 9 à 12, sur la peau, dans lequel le cosmétique le traitement et/ou le soin non thérapeutique de la peau est : l'amélioration et/ou l'augmentation de la microcirculation ou de la vascularisation cutanée ; l'amélioration ou l'augmentation de l'oxygénation de la peau ; l'amélioration ou l'augmentation de la rosée de la peau (rougeur naturelle) ; la réduction et/ou la prévention des symptômes du vieillissement cutané ; l'augmentation de la douceur de la peau ; et/ou l'amélioration ou l'augmentation de la brillance et/ou de la luminosité de la peau.
